# EUROPÄISCHE PATENTSCHRIFT

(11) **EP 1 694 725 B1**
(45) Veröffentlichungstag und Bekanntmachung des Hinweises auf die Patenterteilung: **02.09.2009**
(21) Anmeldenummer: 04803649.5
(22) Anmeldetag: 08.12.2004
(51) Int. Cl.: C08F 220/00

(54) **ANIONISCHE AMPHOLYTISCHE COPOLYMERE**
AMPHOLYTIC ANIONIC COPOLYMERS
COPOLYMERES ANIONIQUES AMPHOLITIQUES

(30) Priorität: 09.12.2003 DE 10357487
(43) Veröffentlichungstag der Anmeldung: 30.08.2006
(73) Patentinhaber: BASF SE, 67056 Ludwigshafen (DE)
(72) Erfinder: NGUYEN-KIM, Son, 69502 Hemsbach (DE); MATHAUER, Klemens, 69115 Heidelberg (DE); WOOD, Claudia, 69469 Weinheim (DE); SCHUH, Gerd, 67365 Schwegenheim (DE); PATWARDHAN, Darshan, 67141 Neuhofen (DE)
(74) Vertreter: Reitstötter - Kinzebach
(86) Internationale Anmeldenummer: PCT/EP2004/013983
(87) Internationale Veröffentlichungsnummer: WO 2005/058988

(56) Entgegenhaltungen:
- WO-A-01/62809
- WO-A-20/04058837
- US-A- 3 927 199

## Beschreibung

Die vorliegende Erfindung betrifft ampholytische Copolymere, die einen molaren Überschuss an anionogenen und/oder anionischen Gruppen enthalten, Polyelektrolytkomplexe, die solch ein ampholytisches Copolymer enthalten, kosmetische und pharmazeutische Mittel, die wenigstens ein solches Copolymer oder Polyelektrolytkomplex enthalten sowie die Verwendung dieser Copolymere und Polyelektrolytkomplexe.

Polymere mit einer größeren Anzahl ionisch dissoziierbarer Gruppen in der Hauptkette und/oder einer Seitenkette werden als Polyelektrolyte bezeichnet. Weisen diese Polymere sowohl anionogene/anionische als auch kationogene/kationische Gruppen auf, so handelt es sich um amphotere Polyelektrolyte bzw. ampholytische Polymere. Diese können entgegengesetzt geladene/ladbare Gruppen in äquimolaren Mengen oder mit einem molaren Überschuss einer der Spezies enthalten. So steht der Ausdruck "anionische ampholytische Copolymere" für ampholytische Copolymere, die einen molaren Überschuss an anionogenen und/oder anionischen Gruppen enthalten. Ein ionogenes bzw. ionisches Polymer kann mit einem gegensätzlich ladbaren bzw. geladenen Polymer unter Ausbildung eines Polyelektrolyt-Komplexes-(Symplexes) reagieren. Ampholytische Polymere können dabei prinzipiell mit wenigstens einem weiteren anionogenen/anionischen, kationogenen/kationischen und/oder ampholytischen Polymer derartige Polyelektrolyt-Komplexe ausbilden. Polyelektrolyte mit ausreichender Anzahl dissoziierbarer Gruppen sind wasserlöslich oder wasserdispergierbar und haben vielfältige Anwendungen im Bereich der Anstrichmittel, Papierhilfsmittel, bei der Textilherstellung sowie speziell in der Pharmazie und Kosmetik gefunden.

Kosmetisch und pharmazeutisch akzeptable wasserlösliche Polymere dienen beispielsweise in Seifen, Cremes und Lotionen als Formulierungsmittel, z. B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen.

Für die Haarkosmetik werden filmbildende Polymere beispielsweise als Conditioner dazu eingesetzt, um die Trocken- und Nasskämmbarkeit, Anfassgefühl, Glanz und Erscheinungsform zu verbessern sowie dem Haar antistatische Eigenschaften zu verleihen. Es ist bekannt, wasserlösliche Polymere mit kationischen Funktionalitäten in Haarkonditioniermitteln einzusetzen, die eine größere Affinität zur strukturell bedingt negativ geladenen Oberfläche des Haares aufweisen und eine elektrostatische Aufladung des Haares verhindern. Struktur und Wirkungsweise verschiedener Haarbehandlungspolymere sind in Cosmetic & Toiletries 103 (1988) 23 beschrieben. Handelsübliche kationische Conditionerpolymere sind z. B. kationische Hydroxyethylcellulose, kationische Polymere auf der Basis von N-Vinylpyrrolidon, z. B. Copolymere aus N-Vinylpyrrolidon und quartemiertem N-Vinylimidazol oder Copolymere aus Acrylamid und Diallyldimethylammoniumchlorid.

Für die Haarkosmetik werden filmbildende Polymere weiterhin als Festigerharze eingesetzt, um der Frisur Halt zu verleihen. Anforderungen an Festigerharze sind zum Beispiel eine starke Festigung bei hoher Luftfeuchtigkeit, Elastizität, Auswaschbarkeit vom Haar, Verträglichkeit in der Formulierung und ein angenehmer Griff des damit behandelten Haares. Zur Festigung von Haarfrisuren werden beispielsweise Vinyllactam-Homo- und Copolymere und Carboxylatgruppen-haltige Polymere eingesetzt.

Schwierigkeiten bereitet oft die Bereitstellung von Produkten mit einem komplexen Eigenschaftsprofil. So besteht ein Bedarf an Polymeren für haarkosmetische Mittel, die zur Bildung im Wesentlichen glatter, klebfreier Filme befähigt sind, die gegenüber dem Haar eine gute Festigungswirkung (auch bei hoher Luftfeuchtigkeit) aufweisen und dem Haar gleichzeitig gute sensorisch erfassbare Eigenschaften, wie Elastizität und einen angenehmen Griff, verleihen. Sollen diese Polymere in Haarsprayformulierungen eingesetzt werden, so ist zudem eine gute Treibgasverträglichkeit, die Eignung für einen Einsatz in low-VOC-Formulierungen, eine gute Löslichkeit in Wasser oder wässrig/alkoholischen Lösungsmittelgemischen und eine gute Auswaschbarkeit erwünscht.

In vielen Fällen lässt sich das gewünschte Eigenschaftsprofil nur durch Einsatz mehrerer kosmetisch aktiver Komponenten, beispielsweise mehrerer Polymere mit ionischen Gruppen erzielen. Dabei zeigt sich jedoch häufig eine Unverträglichkeit der verschiedenen Komponenten miteinander, was beispielsweise dazu führen kann, dass sich keine klaren Formulierungen mehr herstellen lassen. Der Einsatz mehrerer miteinander nicht ausreichend verträglicher Polyelektrolyte kann zu einem unerwünschten Aussalzen führen. Es besteht daher Bedarf an kosmetisch und pharmazeutisch verträglichen Polyelektrolyten, die bei einem Einsatz als einzige Polymerkomponente geeignet sind, ein bestimmtes Eigenschaftsprofil bereitzustellen und/oder die mit einer Vielzahl verschiedener Komponenten, insbesondere Polyelektrolyten, verträglich sind.

Es ist bekannt, Polymere auf Basis von tert.-Butyl(meth)acrylat in Haarkosmetika einzusetzen. So beschreibt die EP-A-0 257 444 Terpolymere aus tert.-Butyl(meth)acrylat, Vinylpyrrolidon und (Meth)acrylsäure und diese enthaltende haarkosmetische Zusammensetzungen. Die WO 94/24986 beschreibt ein Haarfestigungsmittel, das als Filmbildner ein Copolymerisat enthält, das tert.-Butyl(meth)acrylat, (Meth)acrylsäure und gegebenenfalls weitere Monomere einpolymerisiert enthält, wobei mindestens eines der zusätzlichen Monomere ein Homopolymerisat mit einer Glasübergangstemperatur von kleiner als 30 °C liefert.

Die EP-A-183 466 beschreibt ein Verfahren zur Herstellung einer Polymerdispersion durch Polymerisation eines wasserlöslichen Monomers in einem wässrigen salzhaltigen Medium in Gegenwart eines Dispergiermittels. Bei dem wasserlöslichen Monomer kann es sich u. a. um eine ethylenisch ungesättigte Verbindung mit einer kationischen oder anionischen Gruppe handeln. Als Dispergiermittel können Polyelektrolyte eingesetzt werden, deren ionogene/ionische Gruppen der Ladung der eingesetzten Monomere entsprechen müssen.

Die EP-A-670 333 beschreibt vernetzte wasserlösliche Polymerdispersionen, die durch Polymerisation eines Monomergemischs, enthaltend wenigstens ein wasserlösliches Monomer, wenigstens einen Vernetzer sowie gegebenenfalls hydrophobe und/oder amphiphile Monomere in Gegenwart eines polymeren Dispergiermittels erhältlich sind. Als wasserlösliche Monomere können neben einer Vielzahl weiterer auch N-Vinylpyrrolidon sowie Monomere mit kationischen/kationisierbaren Gruppen, wie N-Vinylimidazol, eingesetzt werden. Bei den polymeren Dispergiermitteln kann es sich um Polyelektrolyte handeln, die beispielweise Salze der (Meth)acrylsäure als anionische Monomerbausteine oder quaternierte Derivate von N,N-Dimethylaminoethyl(meth)acrylat als kationische Bausteine einpolymerisiert enthalten. Ein Einsatz der Polymerdispersionen in der Kosmetik wird nicht beschrieben.

Die WO 00/39176 beschreibt ein hydrophiles kationisches ampholytisches Copolymer, das 0,05 bis 20 Mol-% eines anionischen Monomers mit wenigstens einer Carboxygruppe und 10 bis 45 Mol-% eines kationischen Monomers mit wenigstens einer Aminogruppe einpolymerisiert enthält, wobei das Molverhältnis von kationischern zu anionischem Monomer etwa 2 : 1 bis 16 : 1 beträgt. Diese ampholytischen Copolymere können unter Anderem zur Modifizierung der rheologischen Eigenschaften von Körperpflegemitteln eingesetzt werden.

Die WO 02/41856 beschreibt die Verwendung von Polymerdispersionen, die durch Polymerisation wenigstens eines wasserlöslichen Monomers in einer wässrigen Salzlösung, die wenigstens einen Polyelektrolyten als Dispergiermittel enthält, erhältlich sind zur kosmetischen Behandlung von keratinischen Materialien. Zusätzlich enthalten die Dispersionen wenigstens ein Mittel zur Einstellung der Viskosität, beispielsweise eine Polycarbonsäure oder ein Salz davon. Als wasserlösliche Monomere können kationische, anionische und nichtionische Monomere eingesetzt werden, bevorzugt sind Monomergemische, die wenigstens ein kationisches Monomer sowie gegebenenfalls zusätzlich Acrylsäure und/oder Acrylamid enthalten.

Die WO 02/083085 beschreibt eine kosmetische Zusammensetzung, enthaltend eine Dispersion eines kationischen, anionischen oder nichtionischen Polymers in einer wässrigen Salzlösung.

Die EP-A-1038891 beschreibt wasserlösliche oder wasserdispergierbare polymere Salze aus wenigstens einem Polymer und wenigstens einem entgegengesetzt geladenen Neutralisationsmittel.

Die WO 01/62809 beschreibt ein kosmetisches Mittel, das wenigstens ein wasserlösliches oder wasserdispergierbares Polymer enthält, dass
a) 5 bis 50 Gew.-% wenigstens eines α,β-ethylenisch ungesättigten Monomers mit einer tert.-Butylgruppe,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül, und
d) 0 bis 30 Gew.-% wenigstens einer weiteren α,β-ethylenisch ungesättigten Verbindung, wobei es sich um Verbindungen mit mindestens einer anionogenen und/oder anionischen Gruppe pro Molekül handeln kann,
eingebaut enthält.

Die US 3,927,199 beschreibt eine Haarfestigerzusammensetzung, die ein filmbildendes Binderharz auf Basis eines Copolymers enthält, das 1) N-Alkylacrylamide oder methacrylamide, 2) Säuregruppen-haltige Monomere und 3) wenigstens ein weiteres Comonomer einpolymerisiert enthält.

Die US 4,237,253 beschreibt Copolymere für Haarbehandlungsmittel, die 22 bis 64 Mol-% N,N-Dimethylamino-2-ethylmethacrylat, 13 bis 71 Mol-% Methylmethacrylat, 6 bis 23 Mol-% Methacrylsäure und bis zu 22 Mol-% weitere Monomere einpolymerisiert enthalten.

Die WO 95/35087 beschreibt ein amphoteres Haarfestigerpolymer für die Verwendung in Haarsprays und Gelen, das 40 bis 90 Gew.-% eines Hydroxylgruppen-haltigen Monomers, 1 bis 20 Gew.-% eines Säuregruppen-haltigen Monomers und 1 bis 20 Gew.-% eines Amingruppen-haltigen Monomers einpolymerisiert enthält.

Die unveröffentlichte deutsche Patentanmeldung P 102 61 750.3 beschreibt ein ampholytisches Copolymer, das durch radikalische Copolymerisation von
a) wenigstens einer ethylenisch ungesättigten Verbindung mit mindestens einer anionogenen und/oder anionischen Gruppe,
b) wenigstens einer ethylenisch ungesättigten Verbindung mit mindestens einer kationogenen und/oder kationischen Gruppe,
c) wenigstens einer ungesättigten amidgruppenhaltigen Verbindung
sowie gegebenenfalls weiterer Comonomere erhältlich sind. Beschrieben sind weiterhin Polyelektrolyt-Komplexe, die ein solches ampholytisches Copolymer enthalten sowie kosmetisch oder pharmazeutische Mittel auf Basis dieser ampholytischen Copolymere und Polyelektrolyt-Komplexe.

Die unveröffentlichte deutsche Patentanmeldung 102 37 378.7 beschreibt die Verwendung von Polymeren, die erhältlich sind durch
(i) radikalisch initiierte Copolymerisation von Monomergemischen aus
   (a) mindestens einem kationischen Monomeren oder quatemisierbaren Monomeren,
   (b) gegebenenfalls einem wasserlöslichen Monomeren,
   (c) gegebenenfalls einem weiteren radikalisch copolymerisierbaren Monomeren,
   (d) mindestens einem als Vernetzer wirkenden Monomeren mit mindestens zwei ethylenisch ungesättigten, nichtkonjugierten Doppelbindungen, und
   (e) mindestens einem Regler,
(ii) anschließende Quatemisierung oder Protonierung des Polymeren, sofern als Monomeres (a) ein nicht oder nur partiell quatemisiertes Monomer eingesetzt wird,
in haarkosmetischen Zubereitungen.

Die unveröffentlichte deutsche Patentanmeldung 103 31 865.8 beschreibt eine wässrige Polymerdispersion Pd), die erhältlich ist durch radikalische Polymerisation eines Monomergemischs M), enthaltend
a) wenigstens eine α,β-ethylenisch ungesättigte amidgruppenhaltige Verbindung der allgemeinen Formel I 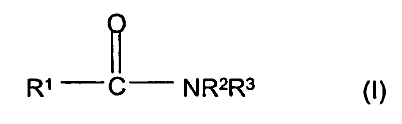 wobei
   R² für eine Gruppe der Formel CH₂=CR⁴- steht und R¹ und R³ unabhängig voneinander für H, Alkyl, Cycloalkyl, Heterocycloalkyl, Aryl oder Hetaryl stehen, oder R¹ und R³ gemeinsam mit der Amidgruppe, an die sie gebunden sind, für ein Lactam mit 5 bis 8 Ringatomen stehen,
b) wenigstens eine radikalisch polymerisierbare vernetzende Verbindung mit wenigstens zwei α,β-ethylenisch ungesättigten Doppelbindungen pro Molekül,
c) wenigstens eine Verbindung mit einer radikalisch polymerisierbaren α,β-ethylenisch ungesättigten Doppelbindung und mindestens einer kationogenen und/oder kationischen Gruppe pro Molekül,

in einem wässrigen Medium in Gegenwart wenigstens eines polymeren anionischen Dispergiermittels D). Sie eignen sich als Konditioniermittel für kosmetische Zubereitungen, insbesondere Shampoos.

Die unveröffentlichte deutsche Patentanmeldung 102 37 378.7 beschreibt ein kosmetisches oder pharmazeutisches Mittel, das wenigstens einen Polyelektrolyt-Komplex umfasst, der als Komponente A1) wenigstens ein wasserlösliches oder wasserdispergierbares Copolymer mit kationogenen Gruppen, das Vinylimidazol und/oder ein Derivat davon und wenigstens ein weiteres damit copolymerisierbares Monomer einpolymerisiert enthält, und als Komponente A2) wenigstens ein Säuregruppen-haltiges Polymer enthält.

Trotz der umfangreichen Bemühungen besteht nach wie vor Verbesserungsbedarf bei den aus dem Stand der Technik bekannten Polymeren zur Erzeugung elastischer Frisuren bei gleichzeitig starker Festigung (auch bei hoher Luftfeuchtigkeit). Für einen erfolgversprechenden Einsatz in Haarsprayformulierungen sind zudem eine gute Treibgasverträglichkeit, eine gute Löslichkeit in Wasser oder wässrig/alkoholischen Lösungsmittelgemischen, die Eignung für einen Einsatz in low-VOC-Formulierungen und eine gute Auswaschbarkeit erwünscht. Gute Eigenschaften sind ebenso bezüglich der Konditionierung des Haars in seinen sensorisch erfassbaren Eigenschaften wie Griff, Volumen, Handhabbarkeit usw. gewünscht. Ferner sollen sich die Polymere durch eine gute Verträglichkeit mit anderen Formulierungsbestandteilen auszeichnen.

Überraschenderweise wurde gefunden, dass sich für die zuvor genannten Anforderungen besonders ampholytische Copolymere eignen, die einen molaren Überschuss an anionogenen und/oder anionischen Gruppen enthalten und die durch radikalische Polymerisation von
a) wenigstens einem verzweigten C₃- bis C₅-Alkylacrylat,
b) Acrylsäure und/oder Methacrylsäure
c) einer Monomerzusammensetzung, enthaltend
   c1) wenigstens eine Verbindung mit einer radikalisch polymerisierbaren, α,β-ethylenisch ungesättigten Doppelbindung und wenigstens einer anionogenen und/oder anionischen Gruppe pro Molekül und
   c2) wenigstens eine Verbindung mit einer radikalisch polymerisierbaren, α,β-ethylenisch ungesättigten Doppelbindung und wenigstens einer kationogenen und/oder kationischen Gruppe pro Molekül,
wobei das molare Verhältnis von anionogenen und anionischen Gruppen der Komponente c1) zu kationogenen und kationischen Gruppen der Komponente c2) etwa 1 : 1 beträgt, erhältlich sind.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die erfindungsgemäßen Copolymere sind im Allgemeinen wasserlöslich.

Zur Einstellung bestimmter Produkteigenschaften der Copolymere können die Monomere a) teilweise durch wenigstens ein Monomer e), wie im Folgenden definiert, ersetzt sein. In einer speziellen Ausführung können die Monomere der Komponente a) dann zu bis zu 50 Gew.-% durch wenigstens ein C₁-C₃-Alkylmethacrylat und/oder Hydroxy-C₁-C₃-alkylmethacrylat ersetzt sein. Geeignete C₁-C₃-Alkylmethacrylate und Hydroxy-C₁-C₃-alkylmethacrylate sind im Folgenden bei der Komponente e) beschrieben. Bevorzugt wird Ethylmethacrylat eingesetzt.

Das zur Herstellung der erfindungsgemäßen Copolymere eingesetzte Monomergemisch weist Monomere mit kationogenen und/oder kationischen Gruppen und Monomere mit anionogenen und/oder anionischen Gruppen auf. Die Menge an zur Polymerisation eingesetzten Monomeren mit anionogenen und/oder anionischen Gruppen wird dabei so bemessen, dass bezogen auf die insgesamt zur Polymerisation eingesetzten Monomere der Molanteil an anionogenen und anionischen Gruppen größer ist als der Molanteil an kationogenen und kationischen Gruppen. Die erfindungsgemäßen Copolymere weisen daher im Mittel einen molaren Überschuss an anionogenen/anionischen Gruppen gegenüber kationogenen/kationischen Gruppen auf. Bevorzugt beträgt das molare Verhältnis von anionogenen/anionischen Gruppen zu kationogenen/kationischen Gruppen wenigstens 1,5 : 1, insbesondere wenigstens 2 : 1.

Überraschenderweise wurde gefunden, dass man Copolymere mit besonders vorteilhaften Eigenschaften erhält, wenn zur Polymerisation die kationogenen/kationischen Monomere c2) gemeinsam mit anionogenen/anionischen Monomeren c1) in Form einer Monomerzusammensetzung, d. h. in Form so genannter "Salzpaare", eingesetzt werden. Dabei liegt das molare Verhältnis von anionogenen und anionischen Gruppen der Komponente c1) zu kationogenen und kationischen Gruppen der Komponente c2) vor zugsweise in einem Bereich von 0,95 : 1 bis 1,05 : 1. Die Salzpaare sind nach außen im Wesentlichen elektroneutral. Als Monomer c1) können in der Monomerzusammensetzung c) auch Acrylsäure und/oder Methacrylsäure eingesetzt werden. Auch dann wird zur Polymerisation zusätzlich weitere Acrylsäure und/oder Methacrylsäure (= Komponente b) eingesetzt.

Zur Herstellung der erfindungsgemäßen Copolymere kann die Acrylsäure bzw. Methacrylsäure b) in teilweise oder vollständig deprotonierter Form eingesetzt werden. Dann leiten sich deren Gegenionen vorzugsweise von Basen ab, wie sie im Folgenden zur Einstellung des pH-Werts bei der Polymerisation oder der erhaltenen Polymerisate beschrieben werden.

Die erfindungsgemäßen Copolymere eignen sich in besonders vorteilhafter Weise für einen Einsatz in kosmetischen Mitteln, insbesondere in Haarbehandlungsmitteln. Sie dienen bevorzugt zur Erzeugung elastischer Frisuren bei gleichzeitig starker Festigung. Vorteilhafterweise zeichnen sie sich zudem sowohl durch eine gute Treibgasverträglichkeit, als auch eine gute Löslichkeit in Wasser oder wässrig/alkoholischen Lösungsmittelgemischen aus. Sie lassen sich somit sowohl zu Haarsprays mit geringem Wassergehalt (VOC mindestens 85 Gew.-%) als auch zu Formulierungen mit hohem Wassergehalt, d.h. geringen VOC-Werten (im Allgemeinen nicht mehr als 55 Gew.%, bezogen auf das Gesamtgewicht des Mittels) formulieren. Dabei zeichnen sich die Haarsprayformulierungen in jedem Fall durch eine sehr gute Sprühbarkeit aus.

### Monomer a)

Die erfindungsgemäßen Copolymere enthalten wenigstens eine Verbindung einpolymerisiert, die vorzugsweise ausgewählt ist unter Isopropylacrylat, sek.-Butylacrylat, Isobutylacrylat, tert.-Butylacrylat, 2-Pentylacrylat, 3-Pentylacrylat, Isopentylacrylat, Neopentylacrylat und Mischungen davon. Besonders bevorzugt ist tert.-Butylacrylat und sind Mischungen, die tert.-Butylacrylat enthalten.

Die erfindungsgemäßen Copolymere enthalten vorzugsweise 20 bis 90 Gew.%, besonders bevorzugt 25 bis 85 Gew.-%, bezogen auf das Gesamtgewicht der zur Polymerisation eingesetzten Monomere, wenigstens eines Monomers a) einpolymerisiert.

Überraschenderweise wurde gefunden, dass Copolymere mit besonders vorteilhaften Eigenschaften erhalten werden, wenn ein Teil der Monomere a) durch wenigstens ein weiteres Monomer, das ausgewählt ist unter tert.-Butylmethacrylat, N-tert.-Octylacrylamid, N-tert.-Butylacrylamid und Mischungen davon, ersetzt werden. Bevorzugt werden höchstens 50 Gew.-%, besonders bevorzugt höchstens 40 Gew.%, insbesondere höchstens 30 Gew.-% der Monomere a) durch wenigstens eines der genannten weiteren Monomere ersetzt. Bevorzugt beträgt der Anteil dieser weiteren Monomere höchstens 30 Gew.%, besonders bevorzugt höchstens 25 Gew.-%, bezogen auf das Gesamtgewicht der zur Herstellung der erfindungsgemäßen Copolymere eingesetzten Monomere. Polymere auf Basis dieser Monomergemische zeichnen sich durch sehr gute Filmbildnereigenschaften aus. Die erhaltenen Filme sind hart, nicht klebrig und gut auswaschbar.

### Monomer b)

Die erfindungsgemäßen Copolymere enthalten vorzugsweise 5 bis 40 Gew.-%, besonders bevorzugt 10 bis 35 Gew.%, insbesondere 13 bis 30 Gew.%, bezogen auf das Gesamtgewicht der zur Polymerisation eingesetzten Monomere, Acrylsäure und/oder Methacrylsäure (=Monomer b) einpolymerisiert.

Überraschenderweise wurde gefunden, dass Copolymere mit besonders vorteilhaften Eigenschaften erhalten werden, wenn ein Teil der Acrylsäure durch Methacrylsäure ersetzt wird. Bevorzugt werden höchstens 65 Gew.-%, besonders bevorzugt höchstens 50 Gew.-% der Acrylsäure durch Methacrylsäure ersetzt. Bevorzugt beträgt der Anteil Methacrylsäure höchstens 20 Gew.%, besonders bevorzugt höchstens 17 Gew.-%, bezogen auf das Gesamtgewicht der zur Herstellung der erfindungsgemäßen Copolymere eingesetzten Monomere. Polymere auf Basis von Acrylsäure/Methacrylsäuregemischen zeichnen sich ebenfalls durch sehr gute Filmbildnereigenschaften aus. Die erhaltenen Filme sind hart, nicht klebrig und gut auswaschbar.

In einer speziellen Ausführung besteht die Komponente b) nur aus Acrylsäure oder nur aus Methacrylsäure.

### Monomerzusammensetzung c)

Die erfindungsgemäßen Copolymere enthalten vorzugsweise 0,5 bis 25 Gew.%, besonders bevorzugt 1 bis 20 Gew.-%, insbesondere 2 bis 16 Gew.%, bezogen auf das Gesamtgewicht der zur Polymerisation eingesetzten Monomere, einer Monomerzusammensetzung c) einpolymerisiert. Die angegebenen Gewichtsanteile beziehen sich sämtlich auf die freie Säureform bzw. die freie Basenform der Monomere c1) und c2).

### Monomer c1)

Die Monomerzusammensetzung c) enthält als Komponente c1) wenigstens eine Verbindung mit einer radikalisch polymerisierbaren, α,β-ethylenisch ungesättigten Doppelbindung und wenigstens einer anionogenen und/oder anionischen Gruppe pro Molekül.

Vorzugsweise sind die Verbindungen c1) ausgewählt unter α,β-ethylenisch ungesättigten Mono- und Dicarbonsäuren, Sulfonsäuren, Phosphonsäuren und Mischungen davon.

Zu den Monomeren c1) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren c1) zählen weiterhin die Halbester von monoethylenisch ungesättigten Dicarbonsäuren, z. B. von Maleinsäure, wie Maleinsäuremonomethylester. Zu den Monomeren c1) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Bevorzugt werden die Monomere c1) in protonierter (nicht geladener) Form zur Bereitstellung der Monomerzusammensetzung c) eingesetzt. Zu den geeigneten Monomeren c1) zählen jedoch auch die Salze der zuvor genannten Säuren, insbesondere die Natrium-, Kalium- und Ammoniumsalze sowie die Salze mit den zuvor genannten Aminen. Die oben angegebenen Gewichtsanteile beziehen sich sämtlich auf die freie Säureform. Die Monomere c1) können in der Monomerzusammensetzung c) als solche oder als Mischungen untereinander eingesetzt werden.

Vorzugsweise ist die Komponente c1) ausgewählt unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon.

Besonders bevorzugt ist die Komponente c1) ausgewählt unter Acrylsäure, Methacrylsäure, Itaconsäure, Crotonsäure und Mischungen davon.

### Monomer c2)

Die Monomerzusammensetzung c) enthält als Komponente c2) wenigstens eine Verbindung mit einer radikalisch polymerisierbaren, α,β-ethylenisch ungesättigten Doppelbindung und wenigstens einer kationogenen und/oder kationischen Gruppe pro Molekül.

Bevorzugt handelt es sich bei den kationogenen bzw. kationischen Gruppen der Komponente c2) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen. Bevorzugt werden die Verbindungen c2) in nicht geladener Form zur Bereitstellung der Monomerzusammensetzung c) eingesetzt. Geeignet ist jedoch auch ein Einsatz in geladener Form. Geladene kationische Gruppen lassen sich z.B. aus den Aminstickstoffen durch Protonierung, z. B. mit einwertigen oder mehrwertigen Carbonsäuren, wie Milchsäure oder Weinsäure, oder Mineralsäuren, wie Phosphorsäure, Schwefelsäure und Salzsäure erzeugen.

Vorzugsweise ist die Komponente c2) ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

Geeignet als Verbindungen c2) sind die Ester von α,β-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Besonders bevorzugt als Verbindungen c2) sind N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Insbesondere werden als Verbindung c2) N-(tert.-Butyl)aminoethylacrylat und N-(tert.-Butyl)aminoethylmethacrylat eingesetzt.

Geeignete Monomere c2) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen. Bevorzugt werden als Monomere c2) N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid und N-[4-(dimethylamino)cyclohexyl]-methacrylamid eingesetzt. Besonders bevorzugt werden N-[3-(dimethylamino)-propyl]acrylamid und/oder N-[3-(dimethylamino)propyl]methacrylamid eingesetzt.

Geeignete Monomere c2) sind weiterhin N,N-Diallylamine und N,N-Diallyl-N-alkylamine und deren Säureadditionssalze. Alkyl steht dabei vorzugsweise für C₁-C₂₄-Alkyl. Bevorzugt ist z.B. N,N-Diallyl-N-methylamin.

Geeignete Monomere c2) sind weiterhin vinyl- und allylsubstituierte Stickstoffheterocyclen, wie N-Vinylimidazol, N-Vinylimidazol-Derivate, z. B. N-Vinyl-2-methylimidazol, vinyl- und allylsubstituierte heteroaromatische Verbindungen, wie 2- und 4-Vinylpyridin, 2- und 4-Allylpyridin, und die Salze davon.

Bevorzugte Monomere c2) sind N-Vinylimidazole der allgemeinen Formel (I), worin R¹ bis R³ für Wasserstoff, C₁-C₄-Alkyl oder Phenyl steht

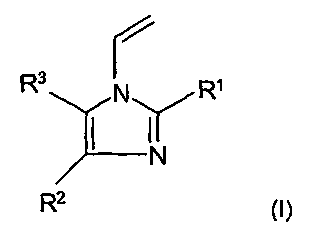

Beispiele für Verbindungen der allgemeinen Formel (I) sind folgender Tabelle 1 zu entnehmen:

**Tabelle 1**

| **R¹** | **R²** | **R³** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Besonders bevorzugt als Komponente c2) sind N-Vinylimidazol und Gemische, die N-Vinylimidazol enthalten. Vorzugsweise enthalten diese Gemische zusätzlich wenigstens eine Verbindung, die ausgewählt ist unter N-(tert.-Butylamino)ethyl(meth)acrylat, N,N-Dimethylaminoethyl(meth)acrylat, N-[3-(dimethylamino)propyl](meth)acrylamid und Mischungen davon.

In einer speziellen Ausführungsform bestehen die erfindungsgemäßen Copolymere nur aus Monomereinheiten der zuvor genannten Monomere a), b) und c). In weiteren Ausführungsformen enthalten die erfindungsgemäßen Copolymere zusätzliche Monomere einpolymerisiert. Geeignete zusätzliche Monomere werden im Folgenden aufgeführt.

### Monomer d)

Bevorzugt enthält das Copolymer zusätzlich wenigstens ein N-Vinyllactam d) einpolymerisiert. Als Monomere d) eignen sich unsubstituierte N-Vinyllactame und N-Vinyllactamderivate, die z. B. einen oder mehrere C₁-C₆-Alkylsubstituenten, wie Methyl, Ethyl, n-Propyl, Isopropyl, n-Butyl, sec.-Butyl, tert.-Butyl etc., aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. Bevorzugt werden N-Vinylpyrrolidon und N-Vinylcaprolactam eingesetzt.

Die erfindungsgemäßen Copolymere enthalten vorzugsweise bis zu 85 Gew.%, besonders bevorzugt bis zu 60 Gew.%, insbesondere bis zu 50 Gew.-%, bezogen auf das Gesamtgewicht der zur Polymerisation eingesetzten Monomere, wenigstens eines N-Vinyllactams d) einpolymerisiert. Wenn ein Monomer d) eingesetzt wird, dann vorzugsweise in einer Menge von mindestens 1 Gew.-%, besonders bevorzugt mindestens 5 Gew.-% und insbesondere wenigstens 10 Gew.-%.

### Monomer e)

Die erfindungsgemäßen Copolymere können zusätzlich wenigstens ein von den Komponenten a) bis d) verschiedenes, damit copolymerisierbares Monomer e) einpolymerisiert enthalten.

Vorzugsweise ist die Komponente e) ausgewählt unter von Komponente a) verschiedenen Estern α,β-ethylenisch ungesättigter Mono- und Dicarbonsäuren mit C₁-C₃₀-Alkanolen und C₁-C₃₀-Alkandiolen, Amiden α,β-ethylenisch ungesättigter Mono- und Dicarbonsäuren mit C₂-C₃₀-Aminoalkoholen, die eine primäre oder sekundäre Aminogruppe aufweisen, N-Vinylamiden gesättigter Monocarbonsäuren, primären Amiden α,β-ethylenisch ungesättigter Monocarbonsäuren und deren N-Alkyl- und N,N-Dialkylderivaten, Estern von Vinylalkohol und Allylalkohol mit C₁-C₃₀-Monocarbonsäuren, Vinylethern, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

Als Monomere e) geeignete N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid.

Geeignete zusätzliche Monomere e) sind weiterhin Acrylsäureamid, Methacrylsäureamid, N-Methyl(meth)acrylamid, N-Ethyl(meth)acrylamid, N-Propyl(meth)acrylamid, N-(n-Butyl)(meth)acrylamid, N-(tert.-Butyl)(meth)acrylamid, N,N-Dimethyl(meth)acrylamid, N,N-Diethyl(meth)acrylamid Piperidinyl(meth)acrylamid und (Meth)acryloylmorpholin.

Geeignete zusätzliche Monomere e) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Geeignete zusätzliche Monomere e) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Geeignete Monomere e) sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20000 auf. Geeignete Polyetherole sind Polyalkylenglykole, wie Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.

Bevorzugt als Komponente e) sind Polyetheracrylate der allgemeinen Formel II 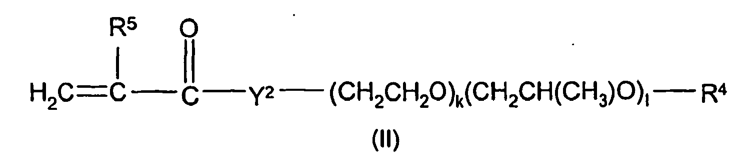 worin
die Reihenfolge der Alkylenoxideinheiten beliebig ist,
- k und: l unabhängig voneinander für eine ganze Zahl von 0 bis 1000 stehen, wobei die Summe aus k und l mindestens 5 beträgt,
- R⁴: für Wasserstoff, C₁-C₃₀-Alkyl oder C₅-C₈-Cycloalkyl steht,
- R⁵: für Wasserstoff oder C₁-C₈-Alkyl steht,
- Y²: für O oder NR⁶ steht, wobei R⁶ für Wasserstoff, C₁-C₃₀-Alkyl oder C₅-C₈-Cycloalkyl steht,

Bevorzugt steht k für eine ganze Zahl von 1 bis 500, insbesondere 3 bis 250. Bevorzugt steht I für eine ganze Zahl von 0 bis 100.

Bevorzugt steht R⁵ für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R⁴ in der Formel II für Wasserstoff, Methyl, Ethyl, n-Propyl, Isopropyl, n-Butyl, sec-Butyl, n-Pentyl, n-Hexyl, Octyl, 2-Ethylhexyl, Decyl, Lauryl, Palmityl oder Stearyl.

Vorzugsweise steht Y² in der Formel II für O oder NH.

Geeignete Polyetheracrylate e) sind z.B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁴-OH hergestellt werden. Die Alkylenoxide können einzeln, alternierend nacheinander oder als Mischung eingesetzt werden. Die Polyetheracrylate e) können allein oder in Mischungen zur Herstellung der erfindungsgemäß eingesetzten Polymere verwendet werden.

Geeignete zusätzliche Monomere e) sind Methyl(meth)acrylat, Methylethacrylat, Ethyl(meth)acrylat, Ethylethacrylat, n-Butyl(meth)acrylat, tert.-Butylmethacrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)äcrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon. Bevorzugte Monomere e) sind die Ester α,β-ethylenisch ungesättigter Mono- und Dicarbonsäuren mit C₁-C₄-Alkanolen.

Geeignete zusätzliche Monomere e) sind weiterhin N-(n-Octyl)(meth)acrylamid, N-(tert.-Octyl)(meth)acrylamid N-(1,1,3,3-Tetramethylbutyl)(meth)acrylamid, N-Ethylhexyl(meth)acrylamid, N-(n-Nonyl)(meth)acrylamid, N-(n-Decyl)(meth)acrylamid, N-(n-Undecyl)(meth)acrylamid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)acrylamid, N-Arrachinyl(meth)acrylamid, N-Behenyl(meth)acrylamid, N-Lignocerenyl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid.

Geeignete zusätzliche Monomere e) sind weiterhin Vinylacetat, Vinylpropionat, Vinylbutyrat und Mischungen davon.

Geeignete zusätzliche Monomere e) sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere e) können einzeln oder in Form von beliebigen Mischungen eingesetzt werden.

Bevorzugt enthalten die erfindungsgemäßen Copolymere wenigstens eine Verbindung e) einpolymerisiert, die ausgewählt ist unter C₁-C₃-Alkylmethacrylaten, Hydroxy-C₁-C₃-alkylmethacrylaten und Mischungen davon. Besonders bevorzugt sind Ethylmethacrylat, Hydroxyethylmethacrylat und Mischungen davon. Insbesondere wird Ethylmethacrylat eingesetzt. Die erfindungsgemäßen Copolymere enthalten diese Monomere vorzugsweise in einer Menge von höchstens 50 Gew.-%, besonders bevorzugt höchstens 45 Gew.%, bezogen auf das Gesamtgewicht aus Verbindungen der Komponente a) und diesen Verbindungen e), einpolymerisiert.

Die erfindungsgemäßen Copolymere enthalten vorzugsweise bis zu 25 Gew.%, besonders bevorzugt bis zu 20 Gew.-%, insbesondere bis zu 15 Gew.-%, bezogen auf das Gesamtgewicht der zur Polymerisation eingesetzten Monomere, wenigstens eines Monomers e) einpolymerisiert. Wenn ein Monomer e) eingesetzt wird, dann vorzugsweise in einer Menge von mindestens 0,1 Gew.%, besonders bevorzugt mindestens 1 Gew.-% und insbesondere wenigstens 5 Gew.-%.

### Vernetzer f)

Die erfindungsgemäßen Copolymere können gewünschtenfalls wenigstens einen Vernetzer, d.h. eine Verbindung mit zwei oder mehr als zwei ethylenisch ungesättigten, nichtkonjugierten Doppelbindungen einpolymerisiert enthalten.

Vorzugsweise werden Vernetzer in einer Menge von 0,01 bis 3 Gew.%, besonders bevorzugt 0,1 bis 2 Gew.%, bezogen auf das Gesamtgewicht der zur Polymerisation eingesetzten Monomere, verwendet.

Geeignete Vernetzer f) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer f) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer f) sind Ester ungesättigter Carbonsäuren mit den oben beschriebenen mehrwertigen Alkoholen, beispielsweise der Ölsäure, Crotonsäure, Zimtsäure oder 10-Undecensäure.

Weitere geeignete Vernetzer f) sind Urethandiacrylate und Urethanpolyacrylate, wie sie z.B. unter der Bezeichnung Laromer® kommerziell erhältlich sind.

Geeignet als Vernetzer f) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer f) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer f) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer f) sind Divinyldioxan, Tetraallylsilan oder Tetravinylsilan.

Selbstverständlich können auch Mischungen der vorgenannten Verbindungen f) eingesetzt werden. Vorzugsweise werden wasserlösliche Vernetzer f) eingesetzt.

Besonders bevorzugt eingesetzte Vernetzer f) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer f) sind Pentaerythrittriallylether, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

Bevorzugt sind Copolymere, die
- 20 bis 90 Gew.%, besonders bevorzugt 25 bis 85, wenigstens einer Verbindung a),
- 5 bis 40 Gew.%, besonders bevorzugt 10 bis 35 Gew.%, insbesondere 13 bis 30 Gew.-%, Acrylsäure und/oder Methacrylsäure b),
- 0,5 bis 25 Gew.%, besonders bevorzugt 1 bis 20 Gew.%, insbesondere 2 bis 16 Gew.-% einer Monomerzusammensetzung c),
- 0 bis 85 Gew.-%, besonders bevorzugt 1 bis 60 Gew.%, insbesondere 5 bis 50 Gew.-% wenigstens einer Verbindung d),
- 0 bis 25 Gew.%, besonders bevorzugt 0,1 bis 20 Gew.%, insbesondere 1 bis 15 Gew.-% wenigstens einer Verbindung e),
- 0 bis 5 Gew.%, besonders bevorzugt 0,01 bis 3 Gew.-%, insbesondere 0,1 bis 2 Gew.-%, wenigstens einer Verbindung f),
einpolymerisiert enthalten.

Eine bevorzugte Ausführung sind Copolymere, die aus Wiederholungseinheiten von
- tert.-Butylacrylat,
- Acrylsäure und/oder Methacrylsäure,
- N-(tert.-Butyl)aminoethyl(meth)acrylat oder N-[3-(dimethylamino)propyl]methacrylamid oder N,N-Dimethylaminoethylmethacrylat oder N-Vinylimidazol
bestehen.

Eine weitere bevorzugte Ausführung sind Copolymere, die aus Wiederholungseinheiten von
- tert.-Butylacrylat,
- tert.-Butylmethacrylat,
- Acrylsäure und/oder Methacrylsäure,
- N-(tert.-Butyl)aminoethyl(meth)acrylat oder N-[3-(dimethylamino)propyl]methacrylamid oder N,N-Dimethylaminoethylmethacrylat oder N-Vinylimidazol
bestehen.

Eine weitere bevorzugte Ausführung sind Copolymere, die aus Wiederholungseinheiten von
- tert.-Butylacrylat,
- Acrylsäure und/oder Methacrylsäure,
- N-(tert.-Butyl)acrylamid und
- N-(tert.-Butyl)aminoethyl(meth)acrylat oder N-[3-(dimethylamino)propyl]methacrylamid oder N,N-Dimethylaminoethylmethacrylat oder N-Vinylimidazol
bestehen.

Eine weitere bevorzugte Ausführung sind Copolymere, die aus Wiederholungseinheiten von
- tert.-Butylacrylat,
- Acrylsäure und/oder Methacrylsäure,
- Vinylpyrrolidon und
- N-(tert.-Butyl)aminoethyl(meth)acrylat oder N-[3-(dimethylamino)propyl]methacrylamid oder N,N-Dimethylaminoethylmethacrylat oder N-Vinylimidazol
bestehen.

Eine weitere bevorzugte Ausführung sind Copolymere, die aus Wiederholungseinheiten von
- tert.-Butylacrylat,
- Acrylsäure und/oder Methacrylsäure,
- N-(tert.-Butyl)acrylamid,
- Vinylpyrrolidon und
- N-(tert.-Butyl)aminoethyl(meth)acrylat oder N-[3-(dimethylamino)propyl]methacrylamid oder N,N-Dimethylaminoethylmethacrylat oder N-Vinylimidazol
bestehen.

Bevorzugt sind weiterhin Copolymere, die
a) tert.-Butylacrylat,
b) Acrylsäure und/oder Methacrylsäure,
c) N-(tert.-Butyl)aminoethylacrylat oder N-(tert.-Butyl)aminoethyl(meth)acrylat oder N-[3-(dimethylaminorpropyl]methacrylamid oder N,N-Dimethylaminoethylmethacrylat oder N-Vinylimidazol,
d) gegebenenfalls Vinylpyrrolidon und
e) wenigstens eine Verbindung, die ausgewählt ist unter C₁-C₃-Alkylmethacrylaten, Hydroxy-C₁-C₃-alkylmethacrylaten und Mischungen davon,
einpolymerisiert enthalten, mit der Maßgabe, dass der Gewichtsmengenanteil der Komponente a) gleich ist wie oder größer ist als der Gewichtsmengenanteil der Komponente e).

Eine besonders bevorzugte Ausführung sind Copolymere, die aus Wiederholungseinheiten von
- tert.-Butylacrylat,
- Acrylsäure und/oder Methacrylsäure,
- N-(tert.-Butyl)aminoethyl(meth)acrylat oder N-Vinylimidazol und
- Ethylmethacrylat

bestehen, mit der Maßgabe, dass der Gewichtsmengenanteil an tert.-Butylacrylat gleich ist wie oder größer ist als der Gewichtsmengenanteil an Ethylmethacrylat.

Eine weitere bevorzugte Ausführung sind Copolymere, die aus Wiederholungseinheiten von
- tert.-Butylacrylat,
- Acrylsäure und/oder Methacrylsäure,
- N-Vinylpyrrolidon,
- N-(tert.-Butyl)aminoethyl(meth)acrylat oder N-Vinylimidazol und
- Ethylmethacrylat
bestehen, mit der Maßgabe, dass der Gewichtsmengenanteil an tert.-Butylacrylat gleich ist wie oder größer ist als der Gewichtsmengenanteil an Ethylmethacrylat.

Bevorzugt sind Copolymere, die, jeweils bezogen auf das Gesamtgewicht der zur Polymerisation eingesetzten Monomere,
- 20 bis 80 Gew.%, bevorzugt 25 bis 75 Gew.-%, tert.-Butylacrylat und Ethylmethacrylat, mit der Maßgabe, dass der Gewichtsmengenanteil an tert.-Butylacrylat gleich ist wie oder größer ist als der Gewichtsmengenanteil an Ethylmethacrylat,
- 5 bis 30 Gew.-%, bevorzugt 10 bis 25 Gew.%, Acrylsäure und/oder Methacrylsäure,
- 1 bis 20 Gew.%, bevorzugt 3 bis 15 Gew.%, einer Monomerzusammensetzung aus Methacrylsäure und N-Vinylimidazol oder N-(tert.-Butyl)aminoethyl(meth)acrylat und
- 0 bis 35 Gew.-% Vinylpyrrolidon
einpolymerisiert enthalten.

Die Herstellung der erfindungsgemäßen Copolymere kann z.B. durch Lösungs-, Fällungs-, Suspensions- oder Emulsionspolymerisation erfolgen. Derartige Verfahren sind dem Fachmann prinzipiell bekannt. Bevorzugt ist die Herstellung durch Lösungspolymerisation.

Erfindungsgemäß wird zur Herstellung der Copolymere eine Monomerzusammensetzung c) eingesetzt, die
c1) wenigstens eine Verbindung mit einer radikalisch polymerisierbaren, α,β-ethylenisch ungesättigten Doppelbindung und wenigstens einer anionogenen und/oder anionischen Gruppe pro Molekül und
c2) wenigstens eine Verbindung mit einer radikalisch polymerisierbaren, α,β-ethylenisch ungesättigten Doppelbindung und wenigstens einer kationogenen und/oder kationischen Gruppe pro Molekül,
enthält, wobei das molare Verhältnis von anionogenen und anionischen Gruppen der Komponente c1) zu kationogenen und kationischen Gruppen der Komponente c2) etwa 1 : 1 beträgt.

Ein Verfahren zur Herstellung eines ampholytischen Copolymers, das einen molaren Überschuss an anionogenen und/oder anionischen Gruppen enthält, beinhaltet, daß man
i) eine Monomerzusammensetzung c) bereitstellt, die
   c1) wenigstens eine Verbindung mit einer radikalisch polymerisierbaren, α,β-ethylenisch ungesättigten Doppelbindung und wenigstens einer anionogenen und/oder anionischen Gruppe pro Molekül und
   c2) wenigstens eine Verbindung mit einer radikalisch polymerisierbaren, α,β-ethylenisch ungesättigten Doppelbindung und wenigstens einer kationogenen und/oder kationischen Gruppe pro Molekül,
   enthält, wobei das molare Verhältnis von anionogenen und anionischen Gruppen der Komponente c1) zu kationogenen und kationischen Gruppen der Komponente c2) etwa 1 : 1 beträgt, und
ii) die Monomerzusammensetzung c) in einer Reaktionszone mit
   a) wenigstens einem verzweigten C₃- bis C₅-Alkylacrylat,
   b) Acrylsäure und/oder Methacrylsäure
   und gegebenenfalls weiteren damit copolymerisierbaren Verbindungen copolymerisiert.

Bezüglich der zur Copolymerisation eingesetzten Komponenten wird auf die vorherigen Ausführungen zu geeigneten und bevorzugten Komponenten der erfindungsgemäßen Copolymere Bezug genommen.

Bevorzugte Lösemittel zur Polymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan. Besonders bevorzugt ist die Polymerisation in Wasser oder einem Wasser/Alkohol-Gemisch, beispielsweise in einem Wasser/Ethanol-Gemisch.

Die Polymerisation kann prinzipiell bei dem durch die eingesetzten Monomere resultierenden pH-Wert erfolgen. Wird zur Polymerisation wenigstens ein N-Vinyllactam eingesetzt (= Komponente d)), so wird der pH-Wert des Polymerisationsmediums vorzugsweise auf einen Wert von 5 bis 8, bevorzugt 6 bis 7, eingestellt. Es ist vorteilhaft, den pH-Wert während der Polymerisation dann in diesem Bereich zu halten. Zur Einstellung des pH-Werts vor, während oder nach der Polymerisation eignen sich prinzipiell alle anorganischen oder organischen Basen (und gegebenenfalls Säuren), insbesondere solche, die außer einer etwaigen Salzbildung keine Reaktion mit den Monomeren eingehen. Geeignete Basen sind z. B. Alkali- und Erdalkalihydroxide, tertiäre Amine, wie Triethylamin, sowie Aminoalkohole, wie Triethanolamin, Methyldiethanolamin oder Dimethylethanolamin. Bevorzugt wird zur Einstellung des pH-Werts wenigstens ein tertiären Amin, das insbesondere ausgewählt ist unter N,N-Dimethylethanolamin, N-Methyldiethanolamin, Triethanolamin und Mischungen davon, eingesetzt. Wird zur Polymerisation wenigstens ein N-Vinyllactam eingesetzt (= Komponente d)), so wird der pH-Wert des Polymerisationsmediums vorzugsweise mit N,N-Dimethylethanolamin eingestellt.

Die Polymerisationstemperaturen liegen vorzugsweise in einem Bereich von etwa 30 bis 120 °C, besonders bevorzugt 40 bis 100 °C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Copolymerisation können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2' - Azobis(2-amidinopropan)hydrochloride (V50 von Wako Pure Chemicals Industries, Ltd.), oder 2,2'-Azobis(2-methyl-butyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu'.

Zur Einstellung des Molekulargewichts kann die Polymerisation in Gegenwart wenigstens eines Reglers erfolgen. Als Regler können die üblichen, dem Fachmann bekannten Verbindungen, wie z.B. Schwefelverbindungen, z.B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden.

Wird bei der Herstellung der Polymere ein organisches Lösungsmittel eingesetzt, so kann dieses durch übliche, dem Fachmann bekannte Verfahren, z. B. durch Destillation bei vermindertem Druck, entfernt werden.

Die erhaltenen flüssigen Polymerzusammensetzungen können durch verschiedene Trocknungsverfahren, wie z. B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall.

### Polymerkomplex PE)

Ein weiterer Gegenstand der Erfindung sind Polyelektrolytkomplexe, die wenigstens ein ampholytisches Copolymer, wie zuvor definiert, und wenigstens einen davon verschiedenen Polyelektrolyten PE) enthalten. Geeignete Polyelektrolyte PE) sind ausgewählt unter anionischen, kationischen und amphoteren Polymeren.

Die Polyelektrolyt-Komplexe enthalten vorzugsweise das ampholytische Copolymer und das Polymer PE) in einem Gewichtsmengenverhältnis von etwa 50:1 bis 1:50, besonders bevorzugt von 20:1 bis 1:20 insbesondere von 5:1 bis 1:5.

Bevorzugt umfassen die erfindungsgemäßen Polyelektrolytkomplexe wenigstens ein Säuregruppen-haltiges Polymer PE).

Geeignete Carbonsäuregruppen-haltige Polymere PE) sind z. B. durch radikalische Polymerisation α,β-ethylenisch ungesättigter Monomere erhältlich. Dabei werden Monomere pe.1) eingesetzt, die wenigstens eine radikalisch polymerisierbare, α,β-ethylenisch ungesättigte Doppelbindung und wenigstens eine anionogene und/oder anionische Gruppe pro Molekül enthalten.

Geeignete Carbonsäuregruppen-haltige Polymere PE) sind weiterhin Carbonsäuregruppen-haltige Polyurethane.

Vorzugsweise sind die Monomere pe.1) ausgewählt unter monoethylenisch ungesättigten Carbonsäuren, Sulfonsäuren, Phosphonsäuren und Mischungen davon.

Zu den Monomeren pe.1) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren pe.1) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren pe. 1) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren pe. 1) zählen auch die Salze der zuvor genannten Säuren, insbesondere die Natrium-, Kalium- und Ammoniumsalze sowie die Salze mit den zuvor genannten Aminen. Die Monomere pe.1) können als solche oder nomere pe. 1) können als solche oder als Mischungen untereinander eingesetzt werden. Die angegebenen Gewichtsanteile beziehen sich sämtlich auf die Säureform.

Vorzugsweise ist die Komponente pe.1) ausgewählt unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon.

Besonders bevorzugt ist die Komponente pe.1) ausgewählt unter Acrylsäure, Methacrylsäure und Mischungen davon.

Die zuvor genannten Monomere pe.1) können jeweils einzeln oder in Form von beliebigen Mischungen eingesetzt werden.

Prinzipiell eignen sich als Comonomere zur Herstellung der Säuregruppen-haltigen Polymere PE) die zuvor als Komponenten des ampholytischen Copolymers genannten Verbindungen a) bis f) mit der Maßgabe, dass der Molanteil an anionogenen und anionischen Gruppen, die das Polymer PE) einpolymerisiert enthält, größer ist als der Molanteil an kationogenen und kationischen Gruppen.

In einer bevorzugten Ausführung enthalten die Polymere PE) wenigstens ein Monomer einpolymerisiert, das ausgewählt ist unter den zuvor genannten Vernetzern f). Auf geeignete und bevorzugte Vernetzer f) wird Bezug genommen.

Als Polymere PE) bevorzugte anionische Polymere sind beispielsweise Homo- und Copolymerisate von Acrylsäure und Methacrylsäure und deren Salze. Dazu zählen auch vernetzte Polymere der Acrylsäure, wie sie unter dem INCI-Namen Carbomer erhältlich sind. Derartige vernetzte Homopolymere der Acrylsäure sind beispielsweise kommerziell unter dem Namen Carbopol® von der Firma BF GOODRICH erhältlich. Bevorzugt sind auch hydrophob modifizierte vernetzte Polyacrylat Polymere, wie Carbopol® Ultrez 21 von der Firma Noveon.

Polyelektrolyt-Komplexe auf Basis von Homo- und Copolymerisaten von Acrylsäure und Methacrylsäure eignen sich in vorteilhafter Weise zur Formulierung als Gele, beispielsweise für Festigergele, sowie zur Formulierung von Schäumen.

Weitere Beispiele für geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn® von der Firma Rohm und Haas erhältlich sind. Besonders geeignete Polymere sind weiterhin Copolymere aus t-Butylacrylat, Ethylacrylat, Methacrylsäure (z. B. Luvimer® 100P), Copolymere aus Ethylacrylat und Methacrylsäure (z. B. Luviumer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset® Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der Meth(acrylsäure), C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester.

Weiterhin umfasst die Gruppe der geeigneten anionischen Polymere beispielhaft Balance® CR (National Starch; Acrylate Copolymer), Balance® 0/55 (National Starch; Acrylate Copolymer), Balance® 47 (National Starch; Octylacrylamid/Acrylate/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP / National Starch; VP/Vinyl Caprolactam/DMAPA Acrylate Copolymer), Allianz® LT-120 (ISP / Rohm & Haas; Acrylat/C1-2 Succinat/Hydroxyacrylat Copolymer), Aquarez® HS (Eastman; Polyester-1), Diaformer® Z-400 (Clariant; Methacryloylethylbetain/Methacrylat-Copolymer), Diaformer® Z-711 (Clariant; Methacryloylethyl N-oxid/Methacrylat-Copolymer), Diaformer® Z-712 (Clariant; Methacryloylethyl N-oxide/Methacrylat-Copolymer), Omnirez® 2000 (ISP; Monoethylester von Poly(Methylvinylether/Maleinsäure in Ethanol), Amphomer® HC (National Starch; Acrylat/ Octylacrylamid-Copolymer), Amphomer® 28-4910 (National Starch; Octylacrylamid/Acrylat/Butylaminoethylmethacrylat-Copolymer), Advantage® HC 37 (ISP; Terpolymer aus Vinylcaprolactam/Vinylpyrrolidon/Dimethylaminoethylmethacrylat), Advantage® LC55 und LC80 oder LC A und LC E, Advantage® Plus (ISP; VA/Butyl Maleate/Isobornyl Acrylate Copolymer), Aculyne® 258 (Rohm & Haas; Acrylat/ Hydroxyesteracrylat-Copolymer), Luviset® P.U.R. (BASF, Polyurethane-1), Luviflex® Silk (BASF), Eastman® AQ 48 (Eastman), Styleze® CC-10 (ISP; VP/DMAPA Acrylates Copolymer), Styleze® 2000 (ISP; VP/Acrylates/Lauryl Methacrylate Copolymer), DynamX (National Starch; Polyurethane-14 AMP-Acrylates Copolymer), Resyn XP (National Starch; Acrylates/Octylacrylamide Copolymer), Fixomer A-30 (Ondeo Nalco; Polymethacrylsäure (und) Acrylamidomethylpropansulfonsäure), Fixate G-100 (Noveon; AMP-Acrylates/Allyl Methacrylate Copolymer).

Geeignete Copolymere PE) sind auch die in der US 3,405,084 beschriebenen Terpolymere aus Vinylpyrrolidon, C₁-C₁₀-Alkyl-Cycloalkyl- und Aryl(meth)acrylaten und Acrylsäure. Geeignete Copolymere PE) sind weiterhin die in der EP-A-0 257 444 und EP-A-0 480 280 beschriebenen Terpolymere aus Vinylpyrrolidon, tert.-Butyl(meth)acrylat und (Meth)acrylsäure. Geeignete Copolymere PE) sind weiterhin die in der DE-A-42 23 066 beschriebenen Copolymere, die wenigstens einen (Meth)acrylsäureester, (Meth)acrylsäure sowie N-Vinylpyrrolidon und/oder N-Vinylcaprolactam einpolymerisiert enthalten. Auf die Offenbarung dieser Dokumente wird hier Bezug genommen.

Geeignete Carbonsäuregruppen-haltige Polymere PE) sind weiterhin Carbonsäuregruppen-haltige Polyurethane.

Die EP-A-636361 offenbart geeignete Blockcopolymere mit Polysiloxanblöcken und Polyurethan-/Polyharnstoffblöcken, die Carbonsäure- und/oder Sulfonsäuregruppen aufweisen. Geeignete siliconhaltige Polyurethane sind auch in der WO 97/25021 und der EP-A-751 162 beschrieben. Geeignete Polyurethane sind auch in der DE-A-42 25 045 beschrieben, worauf hier in vollem Umfang Bezug genommen wird. Diese Polyurethane sind prinzipiell aufgebaut aus
i) mindestens einer Verbindung, die zwei oder mehrere aktive Wasserstoffatome pro Molekül enthält,
ii) mindestens einem Carbonsäuregruppen enthaltenden Diol oder einem Salz davon und
iii) mindestens einem Polyisocyanat.

Bei der Komponente i) handelt es sich z.B. um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Brauchbare Diole i) sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt. Geeignete Aminoalkohole i) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc. Geeignete Diamine i) sind z. B. Ethylendiamin, Propylendiamin, 1,4-Diaminobutan, 1,5-Diaminopentan und 1,6-Diaminohexan sowie α,ω-Diaminopolyether, die durch Aminierung von Polyalkylenoxiden mit Ammoniak herstellbar sind.

Bei der Komponente i) kann es sich auch um ein Polymerisat mit einem zahlenmittleren Molekulargewicht im Bereich von etwa 300 bis 5 000, bevorzugt etwa 400 bis 4 000, insbesondere 500 bis 3 000. Brauchbare Polymerisate i) sind z. B. Polyesterdiole, Polyetherole und Mischungen davon. Polyetherole sind vorzugsweise Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignete Polytetrahydrofurane i) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt. Brauchbare Polyesterdiole i) weisen vorzugsweise ein zahlenmittleres Molekulargewicht im Bereich von etwa 400 bis 5 000, bevorzugt 500 bis 3 000, insbesondere 600 bis 2 000, auf. Als Polyesterdiole i) kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, etc.

Geeignete Verbindungen ii), die zwei aktive Wasserstoffatome und mindestens eine Carbonsäuregruppe pro Molekül aufweisen, sind z. B. Dimethylolpropansäure und Mischungen, die Dimethylolpropansäure enthalten.

Bei der Komponente iii) handelt es sich um übliche aliphatische, cycloaliphatische und/oder aromatische Polyisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat und/oder Dicyclohexylmethandiisocyanat. Gewünschtenfalls können bis zu 3 Mol-% der genannten Verbindungen durch Triisocyanate ersetzt sein.

Geeignete Polymere PE) sind weiterhin kationische Polymere. Dazu zählen z. B. Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Geeignete Polymere PE) sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Die zuvor beschriebenen ampholytischen Copolymere und Polyelektrolytkomplexe eignen sich hervorragend zur Herstellung kosmetischer und pharmazeutischer Mittel. Sie dienen dabei z.B. als polymere Filmbildner in Zubereitungen für die Körperpflege, was die Anwendung in kosmetischen Zubereitungen für keratinöse Oberflächen wie Haut, Haar, Nägel sowie auch Mundpflegepräparate beinhaltet. Sie sind universell in den verschiedensten kosmetischen Zubereitungen einsetzbar und einformulierbar und mit den üblichen Komponenten verträglich. Insbesondere eignen sich die erfindungsgemäßen ampholytischen Copolymere und Polyelektrolytkomplexe zur Herstellung haarkosmetischer Mittel. Gegenüber üblichen, aus dem Stand der Technik bekannten Polymeren eignen sie sich vorteilhaft zur Erzeugung elastischer Frisuren bei gleichzeitig starker Festigung (auch bei hoher Luftfeuchtigkeit). Die erfindungsgemäßen ampholytischen Copolymere und Polyelektrolytkomplexe zeichnen sich zudem durch eine gute Treibgasverträglichkeit, eine gute Löslichkeit in Wasser oder wässrig/alkoholischen Lösungsmittelgemischen, die Eignung für einen Einsatz in low-VOC-Formulierungen und eine gute Auswaschbarkeit aus. Zudem haben sie in der Regel auch gute konditionierende Eigenschaften, d.h. sie verbessern damit behandeltes Haar in seinen sensorisch erfassbaren Eigenschaften wie Griff, Volumen, Handhabbarkeit usw.

### Kosmetisch akzeptabler Träger B)

Die erfindungsgemäßen Mittel weisen einen kosmetisch oder pharmazeutisch akzeptablen Träger B) auf, der ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Die erfindungsgemäßen Mittel weisen z.B. eine Öl- bzw. Fettkomponente B) auf, die ausgewählt ist unter Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestem; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestem, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkemöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden Eigenschaften eignen sich die zuvor beschriebenen Copolymere und Polyelektrolytkomplexe insbesondere als Zusatzstoffe für Haar- und Hautkosmetika.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes Copolymer und/oder einen Polyelektrolytkomplex (= Komponente A), wenigstens einen wie vorstehend definierten Träger B) und wenigstens einen davon verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionem, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebem, Feuchthaltemitteln, Rückfettern, Collagen, Eiweisshydrolysaten, Lipiden, Antioxidantien, Entschäumern, Antistatika, Emollienzien und Weichmachern.

Übliche Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Bevorzugt werden nichtionische Verdicker eingesetzt.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-Bund/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables Polymer enthalten, das sich von den erfindungsgemäßen ampholytischen Copolymeren und den Polymeren unterscheidet, die die erfindungsgemäßen Polyelektrolytkomplexe bilden. Dazu zählen ganz allgemein neutrale Polymere.

Geeignete neutrale Polymere sind z.B. Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Polymere und Polyelektrolytkomplexe geeignet als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und - cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die ampholytischen Copolymere und Polyelektrolytkomplexe verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O-oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen ampholytischen Copolymere und Polyelektrolytkomplexe zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein ampholytischen Copolymere und/oder einen Polyelektrolytkomplexe in einem Anteil von etwa 0,001 bis 30 Gew.%, vorzugsweise 0,01 bis 20 Gew.%, ganz besonders bevorzugt 0,1 bis 12 Gew.-%, bezogen auf das Gesamtgewicht des Mittels.

Besonders Lichtschutzmittel auf Basis der ampholytischen Copolymere und Polyelektrolytkomplexe besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Polyelektrolytkomplexen ampholytischen Copolymere und Polyelektrolytkomplexe und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen ampholytischen Copolymere und Polyelektrolytkomplexe auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem ampholytischen Copolymer und/oder Polyelektrolytkomplex in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein erfindungsgemäßes ampholytisches Copolymere und/oder ein Polyelektrolytkomplexe eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den ampholytischen Copolymeren und Polyelektrolytkomplexen können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein ampholytisches Copolymere und/oder einen Polyelektrolytkomplex sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.%, bevorzugt 5 bis 40 Gew.%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampoo-Formulierungen Verdicker, wie z. B. Kochsalz, PEG-55, Propyleneglykol-Oleat, PEG-120-Methylglucosedioleat und andere, sowie Konservierungsmittel, weitere Wirk- und Hilfsstoffe und Wasser enthalten.

Nach einer besonders bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein ampholytisches Copolymere und/oder einen Polyelektrolytkomplex in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 20 Gew.-% wenigstens eines ampholytischen Copolymers und/ Polyelektrolytkomplexes, wie zuvor definiert,
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0 bis 3 Gew.-% wenigstens eines Verdickers, sowie
f) bis zu 25 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen ampholytischen Copolymere und Polyelektrolytkomplexe eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Spray-Zubereitungen
a) 0,1 bis 10 Gew.-% wenigstens eines ampholytischen Copolymers und/oder Polyelektrolytkomplexes, wie zuvor definiert,
b) 20 bis 99,9 Gew.-% Wasser und/oder Alkohol,
c) 0 bis 70 Gew.-% wenigstens eines Treibmittel,
d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält
a) 0,1 bis 10 Gew.-% wenigstens eines ampholytischen Copolymers und/oder Polyelektrolytkomplexes, wie zuvor definiert,
b) 55 bis 99,8 Gew.-% Wasser und/oder Alkohol,
c) 5 bis 20 Gew.-% eines Treibmittel,
d) 0,1 bis 5 Gew.-% eines Emulgators,
e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Cetheth-1, Polyethylenglycolcetylether; Cetearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quatemium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 10 Gew.-% wenigstens eines ampholytischen Copolymers und/oder Polyelektrolytkomplexes, wie zuvor definiert,
b) 80 bis 99,85 Gew.-% Wasser und/oder Alkohol,
c) 0 bis 3 Gew.-%, bevorzugt 0,05 bis 2 Gew.-%, eines Gelbildners,
d) 0 bis 20 Gew.-% weitere Bestandteile.

Im Allgemeinen wirken die erfindungsgemäßen Polyelektrolytkomplexe bereits "selbstverdickend", so dass in vielen Fällen bei der Herstellung von Gelen auf den Einsatz von Gelbildnern verzichtet werden kann. Ihr Einsatz kann jedoch von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquatemium-7, Polyquaternium-44.

Die erfindungsgemäßen ampholytischen Copolymere und/oder Polyelektrolytkomplexe können in kosmetischen Zubereitungen als Konditioniermittel eingesetzt werden.

Die erfindungsgemäßen ampholytischen Copolymere und/oder Polyelektrolytkomplexe, wie zuvor definiert, können bevorzugt in Shampooformulierungen als Festigungs- und/oder Konditioniermittel eingesetzt werden. Bevorzugte Shampooformulierungen enthalten
a) 0,05 bis 10 Gew.-% wenigstens eines ampholytischen Copolymers und/oder Polyelektrolytkomplexes, wie zuvor definiert,
b) 25 bis 94,95 Gew.-% Wasser,
c) 5 bis 50 Gew.-% Tenside,
c) 0 bis 5 Gew.-% eines weiteren Konditioniermittels,
d) 0 bis 10 Gew.-% weitere kosmetische Bestandteile.

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den ampholytischen Copolymeren und/oder Polyelektrolytkomplexen eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/- Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Ein weiterer Gegenstand der Erfindung ist die Verwendung eines ampholytischen Copolymers und/oder Polyelektrolytkomplexes, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden nicht einschränkenden Beispiele näher erläutert.

### Beispiele

### Allgemeine Herstellungsvorschrift (A): Lösungspolymerisation in Ethanol/Wasser

### Beispiel 15:

600 g einer 30 %igen Polymerlösung (TBA/ TBMAI AS/ DMAPMAM = 65: 10: 22: 3)

### Zulauf 1: Monomerengemisch aus :

| | |
|---|---|
| 117 g | tert.-Butylacrylat |
| 18 g | tert.-Butylmethacrylat |
| 39,6 g | Acrylsäure |
| 5,4 g | Dimethylaminopropylmethacrylamid |
| 147 g | Ethanol |

### Zulauf 2: Initiatorlösung aus:

| | |
|---|---|
| 0,72 g | Wako ® 50 [2,2'-Azobis(2-amindinopropan)dihydrochlorid] |
| 42 g | Wasser |

### Zulauf 3: Initiatorlösung aus:

| | |
|---|---|
| 0,9 g | tert.-Butylperpivalat 75 %ig |
| 31,5 g | Ethanol |

### Zulauf 4: Initiatorlösung aus:

| | |
|---|---|
| 0,9 g | tert.-Butylperpivalat 75 %ig |
| 31,5 g | Ethanol |

### Zulauf 5:

| | |
|---|---|
| 41,6 g | 2-Amino-2-methyl-propanol (AMP) |
| 28 g | Wasser |
| 70 g | Ethanol |

In einer Rührapparatur mit Rückflusskühler, Innenthermometer und vier separaten Zulaufvorrichtungen wurden 16,35 g von Zulauf 1, 2,15g von Zulauf 2, 77 g Wasser und 90 g Ethanol vorgelegt und die Mischung unter Rühren auf ca. 70 °C aufgeheizt. Nach dem Anpolymerisieren, erkennbar an einer leichten Viskositätserhöhung, wurde bei 70 °C der Rest von Zulauf 1 innerhalb von drei Stunden und der Rest von Zulauf 2 innerhalb von vier Stunden zugegeben, wobei die Innentemperatur auf ca. 73 °C erhöht wurde. Die Reaktionslösung wurde noch ca. zwei Stunden bei 70 °C nachgerührt und anschließend der Zulauf 3 innerhalb von 30 Minuten bei 70 °C zudosiert. Nach der Zugabe wurde noch ca. zwei Stunden bei einer Temperatur von 80 °C gerührt. Anschließend wurde der Zulauf 4 innerhalb von 10 Minuten zudosiert und noch ca. zwei Stunden bei einer Temperatur von 80 °C nachpolymerisiert. Die Polymerlösung wurde mit AMP (Zulauf 5, Zugabedauer 10 Minuten) neutralisiert. Man erhielt eine ca. 30 %ige wässrig/ethanolische Lösung.

Analog wurden die Polymere Nr. 1 - 26 und 35 bis 39 hergestellt.

### Allgemeine Herstellungsvorschrift (B):

Lösungspolymerisation in Ethanol/Wasser mit anschließender Wasserdampfdestillation

### Beispiel 29:

900 g einer ca. 20 %igen Polymerlösung (TBA/ VP/ AS/ MAS/ VI = 40: 40: 10: 5: 5)

### Zulauf 1: Monomerengemisch aus :

| | |
|---|---|
| 72 g | tert.-Butylacrylat |
| 72 g | Vinylpyrrolidon |
| 18 g | Acrylsäure |
| 9 g | Methacrylsäure |
| 9 g | Vinylimidazol |
| 15 g | N,N-Dimethylethanolamin |

### Zulauf 2: Initiatorlösung aus:

| | |
|---|---|
| 0,18 g | Wako 50 ® [2,2'-Azobis(2-amindinopropan)dihydrochlorid] |
| 50 g | Wasser |

### Zulauf 3: Initiatorlösung aus:

| | |
|---|---|
| 0,9 g | tert.-Butylperpivalat 75 %ig |
| 63 g | Ethanol |

### Zulauf 4:

| | |
|---|---|
| 360 g | Ethanol |

In einer Rührapparatur mit Rückflusskühler, Innenthermometer und vier separaten Zulaufvorrichtungen wurden 9 g von Zulauf 1, 2,5 g von Zulauf 2, 75 g Wasser und 75 g Ethanol vorgelegt und die Mischung unter Rühren auf ca. 63°C aufgeheizt. Nach dem Anpolymerisieren, erkennbar an einer leichten Viskositätserhöhung, wurde bei 65 °C der Rest von Zulauf 1 innerhalb von drei Stunden und der Rest von Zulauf 2 innerhalb von vier Stunden zugegeben. Die Reaktionslösung wurde noch ca. zwei Stunden bei 65 °C nachgerührt. Der Zulauf 3 wurde bei ca. 75 °C in 30 Minuten zudosiert und die Polymermischung noch ca. zwei Stunden bei 80 °C gerührt. Anschließend wurde bei einer Außentemperatur von 120 °C Ethanol aus der Reaktionslösung durch Wasserdampfdestillation entfernt. Die Polymerlösung wurde auf ca. 40 °C abgekühlt und mit Ethanol (Zulauf 4) verdünnt. Die Polymerlösung wurde mit N,N-Dimethylethanolamin auf pH 8-8,3 und mit Wasser auf einen Feststoffgehalt von 20 % eingestellt. Man erhielt eine klare hellgelbe Lösung.

Analog wurden die Polymere Nr. 27 - 34 und 40 bis 42 hergestellt.

**Tabelle 1**

| Bsp. -Nr. | TBA | OAA | TMBA | NtBAM | VP | AS | MAS | NtBAEMA | VI | DMAP-MAM | Amin/Ng. | K-Wert |
|---|---|---|---|---|---|---|---|---|---|---|---|---|
| 1 | 80 | -- | -- | -- | -- | 18 | -- | -- | 2 | -- | AMP/90 | 41,8 |
| 2 | 80 | -- | -- | -- | -- | 17 | -- | 3 | -- | -- | AMP/90 | 42,7 |
| 3 | 75 | -- | -- | -- | -- | 22 | -- | 3 | -- | -- | AMP/85 | 38,2 |
| 4 | 75 | -- | -- | -- | -- | 20 | -- | -- | -- | 5 | AMP/85 | 39,4 |
| 5 | 75 | -- | -- | -- | -- | 15 | 5 | 5 | -- | -- | AMP/85 | 43,4 |
| 6 | 70 | -- | -- | -- | -- | 17 | 10 | 3 | -- | -- | AMP/85 | 47,8 |
| 7 | 70 | -- | -- | -- | -- | 17 | 10 | -- | -- | 3 | AMP/85 | 45,7 |
| 8 | 70 | -- | -- | -- | -- | 15 | 10 | -- | 5 | -- | AMP/85 | 46,9 |
| 9 | 70 | -- | -- | -- | -- | 14 | 12 | -- | 4 | -- | AMP/85 | 48,1 |
| 10 | 70 | 5 | -- | -- | -- | 22 | -- | 3 | - | -- | TEA/90 | 46,8 |
| 11 | 70 | 5 | -- | -- | -- | 22 | -- | -- | 3 | -- | TEA/90 | 45,1 |
| 12 | 70 | 5 | -- | -- | -- | 22 | -- | -- | -- | 3 | TEA/90 | 47,3 |
| 13 | 65 | -- | 10 | -- | -- | 22 | -- | 3 | -- | -- | AMP/90 | 45,3 |
| 14 | 65 | -- | 10 | -- | -- | 22 | -- | -- | 3 | -- | AMP/90 | 44,6 |
| 15 | 65 | -- | 10 | -- | -- | 22 | -- | -- | -- | 3 | AMP/90 | 46,6 |
| 16 | 60 | -- | 10 | -- | -- | 15 | 10 | -- | -- | 5 | AMP/90 | 51,0 |
| 17 | 67 | -- | -- | 10 | -- | 20 | -- | 3 | -- | -- | AMP/85 | 44,7 |
| 18 | 67 | -- | -- | 10 | -- | 20 | -- | -- | 3 | -- | AMP/85 | 44,1 |
| 19 | 67 | -- | -- | 10 | -- | 20 | -- | -- | -- | 3 | AMP/85 | 43,8 |
| 20 | 60 | -- | - | 12 | -- | 24 | -- | 4 | -- | -- | AMP/85 | 46,4 |
| 21 | 60 | -- | -- | 12 | -- | 24 | -- | -- | 4 | -- | AMP/85 | 47,7 |
| 22 | 60 | -- | -- | 12 | -- | 24 | -- | -- | -- | 4 | AMP/85 | 49,2 |
| 23 | 60 | -- | -- | 15 | -- | 20 | -- | 5 | -- | -- | AMP/85 | 43,6 |
| 24 | 60 | -- | -- | 15 | -- | 20 | -- | -- | 5 | -- | AMP/85 | 45,2 |
| 25 | 60 | -- | -- | 15 | -- | 20 | -- | -- | -- | 5 | AMP/85 | 44,7 |
| 26 | 55 | -- | -- | 20 | -- | 22 | -- | -- | -- | 3 | AMP/85 | 49,6 |
| 27 | 48 | -- | -- | -- | 32 | 17 | -- | -- | 3 | -- | DMEA auf pH 8,0 | 39,5 |
| 28 | 45 | -- | -- | -- | 30 | 10 | 10 | -- | -- | 5 | DMEA auf pH 8,0 | 52,9 |
| 29 | 40 | | -- | -- | 40 | 10 | 5 | -- | 5 | -- | DMEA auf pH 8.0 | 47,0 |
| 30 | 40 | | -- | -- | 40 | 17 | -- | 3 | -- | -- | DMEA auf pH8,0 | 39.6 |
| 31 | 40 | | -- | -- | 40 | 17 | -- | -- | 3 | -- | DMEA auf pH 8,0 | 45,9 |
| 32 | 35 | | -- | -- | 45 | 20 | -- | 5 | -- | -- | DMEA auf pH 8,0 | 45,3 |
| 33 | 35 | | -- | -- | 45 | 20 | -- | -- | 5 | -- | DMEA auf pH 8,0 | 43,8 |
| 34 | 30 | | -- | 20 | 30 | 10 | -- | 10 | -- | -- | DMEA auf pH 8,0 | 42,5 |

**Tabelle 2:**

| Beispiel Nr. | TBA | EMA | VP | AS | MAS | VI | NtBAEMA |
|---|---|---|---|---|---|---|---|
| 35 | 68 | -- | -- | 3 | 22 | 7 | -- |
| 36 | 65 | -- | -- | 5 | 20 | -- | 10 |
| 37 | 44 | 30 | -- | 3 | 20 | 3 | -- |
| 38 | 40 | 30 | -- | 3 | 20 | 7 | -- |
| 39 | 40 | 30 | -- | 5 | 20 | -- | 5 |
| 40 | 50 | -- | 30 | -- | 15 | 5 | -- |
| 41 | 50 | -- | 25 | 3 | 15 | 7 | -- |
| 42 | 45 | -- | 30 | -- | 15 | -- | 10 |

| | | | | | | | |
|---|---|---|---|---|---|---|---|
| TBA tert.-Butylacrylat EMA Ethylmethacrylat OAA N-tert.-Octylacrylamid TBMA tert.-Butylmethacrylat NtBAM N-tert.-Butylacryamid VP Vinylpyrrolidon AS Acrylsäure MAS Methacrylsäure NtBAEMA N-(tert.-Butyl)aminoethylmethacrylat VI Vinylimidazol DMAPMAM Dimethylaminopropylmethacrylamid Amin AMP = 2-Amino-2-methylpropanol TEA = Triethanolamin DMEA = Dimethylethanolamin Ng. Neutralisationsgrad K-Wert 1 %ig in N-Methylpyrrolidon | | | | | | | |

### Anwendungstechnische Beispiele:

### I) Verwendung in der Haarkosmetik:

### 1) VOC 80 Aerosol-Haarspray (Beispiele Nr. 1 - 26)

| | |
|---|---|
| Polymer 1-26 (30 %ige wässrig-ethanol. Lösung) | 10,0 |
| Wasser | 15,0 |
| Dimethylether | 40,0 |
| Ethanol | 35,0 |

| | |
|---|---|
| Weitere Zusatzstoffe: Konservierungsmittel, lösliches ethoxiliertes Silikon, Parfüm, Entschäumer... | |

### 2) VOC 80 Aerosol-Haarspray (Beispiele Nr. 27 - 34)

| | |
|---|---|
| Polymer 27, 28, 29, 30, 31, 32, 33, 34 (20 %ige wässrig-ethanol. Lösung) | 15,0 |
| Wasser | 11,0 |
| Dimethylether | 40,0 |
| Ethanol | 34,0 |

| | |
|---|---|
| Weitere Zusatzstoffe: Konservierungsmittel, lösliches ethoxiliertes Silikon, Parfüm, Entschäumer... | |

### 3) VOC 55 Aerosol-Haarspray (Beispiele Nr. -35 - 65)

| | |
|---|---|
| Polymer 1-26, 35, 36, 37, 38, 39 (30 %ige wässrig-ethanol. Lösung) | 6.67 |
| Wasser | 41,7 |
| Dimethylether | 40,0 |
| Ethanol | 11,7 |

| | |
|---|---|
| Weitere Zusatzstoffe: Konservierungsmittel, lösliches ethoxiliertes Silikon, Parfüm, Entschäumer... | |

### 4) VOC 55 Aerosol-Haarspray (Beispiele Nr. -66 - 76)

| | |
|---|---|
| Polymer 27, 28, 29, 30, 31, 32, 33, 34, 40, 41, 42 (20 %ige wässrig-ethanol. Lösung) | 10,0 |
| Wasser | 39,0 |
| Dimethylether | 40,0 |
| Ethanol | 11,0 |

| | |
|---|---|
| Weitere Zusatzstoffe: Konservierungsmittel, lösliches ethoxiliertes Silikon, Parfüm, Entschäumer... | |

### 5) VOC 55 Aerosol-Haarspray (Beispiele Nr. -77 - 107)

| | |
|---|---|
| Polymer 1-26, 35, 36, 37, 38, 39 (30 %ige wässrig-ethanol. Lösung) | 6,7 |
| Luvimer® Low VOC (neutralisiert mit AMP) | 1,0 |
| Wasser | 40,6 |
| Dimethylether | 40,0 |
| Ethanol | 11,7 |

| | |
|---|---|
| Weitere Zusatzstoffe: Konservierungsmittel, lösliches ethoxiliertes Silikon, Parfüm, Entschäumer... | |

### 6) VOC 55 Aerosol-Haarspray (Beispiele Nr. -108 118)

| | |
|---|---|
| Polymer 27, 28, 29, 30, 31, 32, 33, 34, 40, 41, 42 (20 %ige wässrig-ethanol. Lösung) | 10,0 |
| Luviset PUR (30 %ige Lösung) | 5,0 |
| Wasser | 34,5 |
| Dimethylether | 40,0 |
| Ethanol | 10,5 |

| | |
|---|---|
| Weitere Zusatzstoffe: Konservierungsmittel, lösliches ethoxiliertes Silikon, Parfüm, Entschäumer... | |

### 7) VOC 55 Pumpspray (Beispiele Nr. -119 - 149)

| | |
|---|---|
| Polymer 1-26, 35, 36, 37, 38, 39 (30%ige wässrig-ethanol. Lösung ) | 6,67 |
| Wasser | 41,7 |
| Ethanol | 51,7 |

| | |
|---|---|
| Weitere Zusatzstoffe: Konservierungsmittel, lösliches ethoxiliertes Silikon, Parfüm, Entschäumer... | |

### 8) VOC 55 Pumpspray (Beispiele Nr. -150 - 160)

| | |
|---|---|
| Polymer 27, 28, 29, 30, 31, 32, 33, 34, 40, 41, 42 (20 %ige wässrig-ethanol. Lösung) | 10,0 |
| Luviset ® PUR (30 %ige Lösung) | 5,0 |
| Wasser | 34,5 |
| Ethanol | 50,5 |

| | |
|---|---|
| Weitere Zusatzstoffe: Konservierungsmittel, lösliches ethoxiliertes Silikon, Parfüm, Entschäumer... | |

### 9) Schaumfestiger (Beispiele Nr. -161 -176)

| | |
|---|---|
| Polymer Nr. 3, 4, 5, 6, 7, 8, 9, 13, 20, 21, 22, 35, 36, 37, 38, 39 (30 %ige Lösung) | 5,0 |
| Luviflex ® Soft (10%-ig wässrige Lösung, pH=7) | 15,0 (Acrylat-Copolymer, Fa. BASF) |
| Cremophor ® A 25 | 0,2 (Ceteareth 25, Fa. BASF) |
| Comperlan ® KD | 0,1 (Coamide DEA, Fa. Henkel) |
| Wasser | 69,7 |
| Dimethylether | 10,0 |

| | |
|---|---|
| Weitere Zusatzstoffe: Parfüm, Konservierungsmittel... | |

### Herstellung : Einwiegen und unter Rühren lösen. Abfüllen und Treibgas zusetzen.

### 10) Schaumfestiger (Beispiele Nr. 177 - 183)

| | |
|---|---|
| Polymer Nr. 28, 32, 33, 34, 40, 41, 42 (20 %ige Lösung) | 15,0 |
| Cremophor ® A 25 | 0,2 (Ceteareth 25, Fa. BASF) |
| Comperlan ® KD | 0,1 (Coamide DEA Fa. Henkel) |
| Wasser | 74,7 |
| Dimethylether | 10,0 |

| | |
|---|---|
| Weitere Zusatzstoffe: Parfüm, Konservierungsmittel... | |

### Herstellung : Einwiegen und unter Rühren lösen. Abfüllen und Treibgas zusetzen.

### 11) Haargele (Beispiele Nr. -184 - 225)

| | [ % ] |
|---|---|
| Phase 1: | |
| Polymer 1-26, 35, 36, 37, 38, 39 (30 %ige Lösung) | 10,0 |
| oder Polymer 27, 28, 29, 30, 31, 32, | |
| 33, 34, 40, 41, 42 (20 %ige Lösung) | 15,0 |
| dest. Wasser auf | 49,0 |
| Aminomethylpropanol (38 %ige Lösung) | 1,0 |
| Weitere Zusatzstoffe: | |
| Konservierungsmittel, lösliches ethoxiliertes Silikon, Parfüm... | |

| Phase 2 : | |
|---|---|
| Acrylsäure/Beheneth-25-methacrylat-Copolymere | 50,0 |
| (Aculyn® 28 von der Firma Rohm und Haas, | |
| 1 %ige wässrige Suspension) | |

| | |
|---|---|
| Herstellung : | Die Komponenten der Phase 1 und 2 werden getrennt eingewogen und homogenisiert. Phase 2 wird langsam in Phase 1 eingerührt. Es bildet sich ein klares, festes Gel. |

### II) Verwendung in der Hautkosmetik:

### 12) Standard O/W-Creme (Beispiele Nr. -226 , 237)

| Ölphase : | % | CTFA Name CTFA Name |
|---|---|---|
| Cremophor A6 | 3,5 | Ceteareth-6 (and) Stearyl Alkohol |
| Cremophor A25 | 3,5 | Ceteareth-25 |
| Glycerinmonostearat s.e. | 2,5 | Glyceryl Stearate |
| Paraffinöl | 7,5 | Paraffin Oil |
| Cetylalkohol | 2,5 | Cetyl Alkohol |
| Luvitol EHO | 3,2 | Cetearyl Octanoate |
| Vitamin-E-acetate | 1,0 | Tocopheryl Acetate |
| Nip-Nip | 0,1 | Methyl- und Propyl-4-hydroxybenzoate (7:3) |

| Wasser-Phase: | | |
|---|---|---|
| | % | CTFA Name |
| Polymer Nr. 12,27, 28, 29, 30, 31, 32, 33, | | |
| 34, 40, 41, 42 (20 %ige wässrige Lösung) | 3,0 | |
| Wasser | 74,6 | |
| 1,2-Propylenglykol | 1,5 | Propylene Glycol |
| Germall II | 0,1 | Imidazolidinyl-Urea |

| | |
|---|---|
| Herstellung : | Die Komponenten werden eingewogen und die Öl-Phase und Wasser-Phase getrennt bei einer Temperatur von ca. 80 °C unter Rühren homogenisieren. Die Wasser-Phase wird langsam in die Öl-Phase eingerührt und die Mischung unter Rühren auf Raumtemperatur abgekühlt. |

### 13) Tageslotion (Beispiele Nr. -238 - 249)

| ölphase : | % | CTFA Name |
|---|---|---|
| Cremophor A6 | 1,5 | Ceteareth-6 (and) Stearyl Alkohol |
| Cremophor A25 | 1,5 | Ceteareth-25 |
| Glycerinmonostearat s.e. | 5,0 | Glyceryl Stearate |
| Uvinul MS 40 | 0,5 | Bezophenone-4 |
| Paraffinöl | 3,5 | Paraffin Oil |
| Cetylalkohol | 0,5 | Cetyl Alkohol |
| Luvitol EHO | 10,0 | Cetearyl Octanoate |
| D-Panthenol 50 P | 3,0 | Panthenol und Propylenglykol |
| Vitamin-E-acetate | 1,0 | Tocopheryl Acetate |
| Tegiloxan 100 | 0,3 | Dimethicon |
| Nip-Nip | 0,1 | Methyl- und Propyl-4-hydroxybenzoate (7:3) |

| Wasser-Phase: | % | |
|---|---|---|
| Polymer Nr. 12,27, 28, 29, 30, 31, 32, 33, 34, 40, 41, 42 (20 %ige wässrige Lösung) | 1,5 | |
| Wasser | 70,0 | |
| 1,2-Propylenglykol | 1,5 | Propylene Glycol |
| Germall II | 0,1 | Imidazolidinyl-Urea |

| | |
|---|---|
| Herstellung : | Die Komponenten werden eingewogen und die Öl-Phase und Wasser-Phase getrennt bei einer Temperatur von ca. 80 °C unter Rühren homogenisieren. Die Wasser-Phase wird langsam in die Öl-Phase eingerührt und die Mischung unter Rühren auf Raumtemperatur abgekühlt. |

## Patentansprüche

1. Ampholytisches Copolymer, enthaltend einen molaren Überschuss an anionogenen und/oder anionischen Gruppen, erhältlich durch radikalische Polymerisation von
a) wenigstens einem verzweigten C₃-C₅-Alkylacrylat,
b) Acrylsäure und/oder Methacrylsäure und
c) einer Monomerzusammensetzung, enthaltend
c1) wenigstens eine Verbindung mit einer radikalisch polymerisierbaren, α,β-ethylenisch ungesättigten Doppelbindung und wenigstens einer anionogenen und/oder anionischen Gruppe pro Molekül und
c2) wenigstens eine Verbindung mit einer radikalisch polymerisierbaren, α,β-ethylenisch ungesättigten Doppelbindung und wenigstens einer kationogenen und/oder kationischen Gruppe pro Molekül,
wobei das molare Verhältnis von anionogenen und anionischen Gruppen der Komponente c1) zu kationogenen und kationischen Gruppen der Komponente c2) etwa 1 : 1 beträgt.

2. Copolymer nach Anspruch 1, wobei die Komponente a) tert.-Butylacrylat umfasst.

3. Copolymer nach Anspruch 1, wobei die Komponente b) aus Acrylsäure und Methacrylsäure besteht, mit der Maßgabe, dass der Gewichtsmengenanteil an Acrylsäure gleich ist wie oder größer ist als der Gewichtsmengenanteil an Methacrylsäure.

4. Copolymer nach Anspruch 1, wobei die Komponente b) aus Acrylsäure oder aus Methacrylsäure besteht.

5. Copolymer nach einem der vorhergehenden Ansprüche, wobei die Komponente c1) ausgewählt ist unter α,β-ethylenisch ungesättigten Mono- und Dicarbonsäuren, Sulfonsäuren, Phosphonsäuren und Mischungen davon.

6. Copolymer nach einem der vorhergehenden Ansprüche, wobei die Komponente c1) ausgewählt ist unter Acrylsäure, Methacrylsäure, Itaconsäure und Mischungen davon.

7. Copolymer nach einem der vorhergehenden Ansprüche, wobei die Komponente c2) ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

8. Copolymer nach einem der vorhergehenden Ansprüche, wobei die Komponente c2) ausgewählt ist unter N-Vinylimldazol, N-(tert,-Butylamino)ethyl(meth)acrylat, N,N-Dimethylaminoethyl(meth)acrylat, N-[3-(dimethylamino)propyl]-(meth)acrylamid und Mischungen davon.

9. Copolymer nach einem der vorhergehenden Ansprüche, das zusätzlich als Komponente d) wenigstens ein N-Vinyllactam einpolymerisiert enthält.

10. Copolymer nach einem der vorhergehenden Ansprüche, das zusätzlich wenigstens ein weiteres Monomer e) einpolymerisiert enthält, das ausgewähll ist unter von Komponente a) verschiedenen Estern α,β-ethylenisch ungesättigter Mono- und Dicarbonsäuren mit C₁-C₃₀-Alkanolen und C₁-C₃₀-Alkandiolen, Amiden α,β-Ethylenisch ungesättigter Mono- und Dicarbonsäuren mit C₂-C₃₀-Aminoalkoholen, die eine primäre oder sekundäre Aminogruppe aufweisen, N-Vinylamiden gesättigter Monocarbonsäuren, primären Amiden α,β-ethylenisch ungesättigter Monocarbonsäuren und deren N-Alkyl- und N,N-Dialkylderivaten, Estern von Vinylalkohol und Allylalkohol mit C₁-C₃₀-Monocarbonsäuren, Vinylethern, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

11. Copolymer nach Anspruch 10, wobei die Komponente e) ausgewählt ist unter C₁-C₃-Alkylmethacrylaten, Hydroxy-C₁-C₃-alkylmethacrylaten und Mischungen davon.

12. Copolymer nach Anspruch 11, wobei die Komponente e) Ethylmethacrylat umfasst oder daraus besteht.

13. Copolymer nach einem der vorhergehenden Ansprüche, das
- 20 bis 90 Gew.%, besonders bevorzugt 25 bis 85, wenigstens einer Verbindung a),
- 5 bis 40 Gew.-%, besonders bevorzugt 10 bis 35 Gew.-%, insbesondere 13 bis 30 Gew.-%, Acrylsäure und/oder Methacrylsäure b),
- 0,5 bis 25 Gew,-%, besonders bevorzugt 1 bis 20 Gew.-%, insbesondere 2 bis 16 Ges,-% einer Monomerzusammensetzung c),
- 0 bis 85 Gew.%, besonders bevorzugt 1 bis 60 Gew.%, insbesondere 5 bis 50 Gew.-% wenigstens einer Verbindung d),
- 0 bis 25 Gew.-%, besonders bevorzugt 0,1 bis 20 Gew.-%, insbesondere 1 bis 15 Gew.-% wenigstens einer Verbindung e),
- 0 bis 5 Gew.-%, besonders bevorzugt 0,01 bis 3 Gew.-%, insbesondere 0,1 bis 2 Gew.-%, wenigstens eines Vernetzers f),
einpolymerisiert enthält.

14. Copolymer nach einem der Ansprüche 1 bis 13, das aus Wiederholungseinheiten von
- tert.-Butylacrylat,
- Acrylsäure und/oder Methacrylsäure
- N-(tert.-Butyl)aminoethyl(meth)acrylat oder N-[3-(dimethylamino)propyl]methacrylamid oder N,N-Dimethylaminoethylmethacrylat oder N-Vinylimidazol
besteht.

15. Copolymer nach einem der Ansprüche 1 bis 13, das aus Wiederholungseinheiten von
- tert.-Butylacrylat,
- tert.-Butylmethacrylat,
- Acrylsäure und/oder Methacrylsäure,
- N-(tert.-Butyl)aminoethyl(meth)acrylat oder N-[3-(dimethylamino)propyl]methacrylamid oder N,N-Dimethylaminoethylmethacrylat oder N-Vinylimidazol
besteht.

16. Copolymer nach einem der Ansprüche 1 bis 13, das aus Wiederholungseinheiten von
- tert.-Butylacrylat,
- Acrylsäure und/oder Methacrylsäure,
- N-(tert.-Butyl)acrylamid und
- N-(tert.-Butyl)aminoethyl(meth)acrylat oder N-[3-(dimethylamino)propyl]methacrylamid oder N,N-Dimethylaminoethylmethacrylat oder N-Vinylimidazol
besteht.

17. Copolymer nach einem der Ansprüche 1 bis 13, das aus Wiederholungseinheiten von
- tert.-Butylacrylat,
- Acrylsäure und/oder Methacrylsäure,
- Vinylpyrrolidon und
- N-(tert.-Butyl)aminoethyl(meth)acrylat oder N-[3-(dimethylamino)propyl]methacrylamid oder N,N-Dimethylaminoethylmethacrylat oder N-Vinylimidazol
besteht.

18. Copolymer nach einem der Ansprüche 1 bis 13, das aus Wiederholungseinheiten von
- tert.-Butylacrylat,
- Acrylsäure und/oder Methacrylsäure,
- N-(tert.-Butyl)acrylamid,
- Vinylpyrrolidon und
- N-(tert.-Butyl)aminoethyl(meth)acrylat oder N-[3-(dimethylamino)propyl]methacrylamid oder N,N-Dimethylaminoethylmethacrylat oder N-Vinylimidazol
besteht.

19. Copolymer nach einem der Ansprüche 1 bis 13, das
a) tert.-Butylacrylat,
b) Acrylsäure und/oder Methacrylsäure,
c) N-(tert.-Butyl)aminoethylacrylat oder N-(tert.-Butyl)aminoethyl(meth)acrylat oder N-[3-(dimethylamino)-propyl]methacrylamid oder N,N-Dimethylaminoethylmethacrylat oder N-Vinylimidazol,
d) gegebenenfalls Vinylpyrrolidon und
e) wenigstens eine Verbindung, die ausgewählt ist unter C₁-C₃-Alkylmethacrylaten, Hydroxy-C₁-C₃-alkylmethacrylaten und Mischungen davon,
einpolymerisiert enthält, mit der Maßgabe, dass der Gewichtsmengenanteil der Komponente a) gleich ist wie oder größer ist als der Gewichtsmengenanteil der Komponente e).

20. Copolymer nach einem der Ansprüche 1 bis 13, das aus Wiederholungseinheiten von
- tert.-Butylacrylat,
- Acrylsäure und/oder Methacrylsäure,
- N-(tert.-Butyl)aminoethyl(meth)acrylat oder N-Vinylimidazol und
- Ethylmethacrylat
besteht, mit der Maßgabe, dass der Gewichtsmengenanteil an tert.-Butylacrylat gleich ist wie oder größer ist als der Gewichtsmengenanteil an Ethylmethacrylat.

21. Copolymer nach einem der Ansprüche 1 bis 13, das aus Wiederholungseinheiten von
- tert.-Butylacrylat,
- Acrylsäure und/oder Methacrylsäure,
- N-Vinylpyrrolidon,
- N-(tert.-Butyl)aminoethyl(meth)acrylat oder N-Vinylimidazol und
- Ethylmethacrylat
besteht, mit der Maßgabe, dass der Gewichtsmengenanteil an tert.-Butylacrylat gleich ist wie oder größer ist als der Gewichtsmengenanteil an Ethylmethacrylat.

22. Copolymer nach einem der Ansprüche 1 bis 13, das, jeweils bezogen auf das Gesamtgewicht der zur Polymerisation eingesetzten Monomere,
- 20 bis 80 Gew.-%, bevorzugt 25 bis 75 Gew.-%, tert.-Butylacrylat und Ethylmethacrylat, mit der Maßgabe, dass der Gewichtsmengenanteil an tert.-Butylacrylat gleich ist wie oder größer ist als der Gewichtsmengenanteil an Ethylmethacrylat,
- 5 bis 30 Gew.%, bevorzugt 10 bis 25 Gew.%, Acrylsäure und/oder Methacrylsäure,
- 1 bis 20 Gew.%, bevorzugt 3 bis 15 Gew.%, einer Monomerzusammensetzung aus Methacrylsäure und N-Vinylimidazol oder N-(tert.-Butyl)aminoethyl(meth)acrylat und
- 0 bis 35 Gew.-% Vinylpyrrolidon
einpolymerisiert enthält.

23. Polyelektrolytkomplex, enthaltend wenigstens ein ampholytisches Copolymer, wie in einem der Ansprüche 1 bis 22 definiert, und wenigstens einen davon verschiedenen Polyelektrolyten PE).

24. Kosmetisches oder pharmazeutisches Mittel, enthaltend
A) wenigstens ein ampholytisches Copolymer, wie in einem der Ansprüche 1 bis 22 definiert, oder einen Polyelektrolytkomplex, wie in Anspruch 23 definiert, und
B) wenigstens einen kosmetisch akzeptablen Träger.

25. Mittel nach Anspruch 24, wobei die Komponente B) ausgewählt ist unter
i) Wasser,
ii) wassermischbaren organischen Lösungsmitteln, vorzugsweise C₂-C₄-Alkanolen, insbesondere Ethanol
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen
viii) Treibgasen
und Mischungen davon.

26. Mittel nach einem der Ansprüche 24 oder 25, enthaltend wenigstens einen von den Komponenten A) und B) verschiedenen Zusatzstoff, der ausgewählt ist unter kosmetisch aktiven Wirkstoffen, Emulgatoren, Tensiden, Konservierungsmitteln, Parfümölen, Verdickern, Haarpolymeren, Haar- und Hautconditionem, Pfropfpolymeren, wasserlöslichen oder dispergierbaren silikonhaltigen Polymeren, Lichtschutzmitteln, Bleichmitteln, Gelbildnem, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumem, Antistatika, Emollienzien und Weichmachern.

27. Mittel nach einem der Ansprüche 24 bis 26 in Form eines Gels, Schaums, Sprays, einer Mousse, Salbe, Creme, Emulsion, Suspension, Lotion, Milch oder Paste.

28. Verwendung eines Copolymers, wie in einem der Ansprüche 1 bis 22 definiert, oder eines Polyelektrolytkomplexes, wie in Anspruch 23 definiert, in Hautreinigungsmitteln, Mitteln zur Pflege und zum Schutz der Haut, Nagelpflegemitteln, Zubereitungen für die dekorative Kosmetik und Haarbehandlungsmitteln.

29. Verwendung nach Anspruch 28 in Haarbehandlungsmitteln als Festiger und/oder als Conditioner.

30. Verwendung nach Anspruch 29, wobei das Mittel in Form eines Haargels, Shampoos, Schaumfestigers, Haarwassers, Haarsprays oder Haarschaums vorliegt.

31. Verwendung eines Copolymers, wie in einem der Ansprüche 1 bis 22 definiert, oder eines Polyelektrolytkomplexes, wie in Anspruch 23 definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

## Claims

1. An ampholytic copolymer comprising a molar excess of anionogenic and/or anionic groups, obtainable by free radical polymerization of
a) at least one branched C₃-C₅-alkyl acrylate,
b) acrylic acid and/or methacrylic acid and
c) a monomer composition comprising
c1) at least one compound having an α,β-ethylenically unsaturated double bond capable of free radical polymerization and at least one anionogenic and/or anionic group per molecule and
c2) at least one compound having an α,β-ethylenically unsaturated double bond capable of free radical polymerization and at least one cationogenic and/or cationic group per molecule,
the molar ratio of anionogenic and anionic groups of component c1) to cationogenic and cationic groups of component c2) being about 1 : 1.

2. The copolymer according to claim 1, the component a) comprising tert-butyl acrylate.

3. The copolymer according to claim 1, the component b) consisting of acrylic acid and methacrylic acid, with the proviso that the proportion by weight of acrylic acid is equal to or greater than the proportion by weight of methacrylic acid.

4. The copolymer according to claim 1, the component b) consisting of acrylic acid or methacrylic acid.

5. The copolymer according to any of the preceding claims, the component c1) being selected from α,β-ethylenically unsaturated mono- and dicarboxylic acids, sulfonic acids, phosphonic acids and mixtures thereof.

6. The copolymer according to any of the preceding claims, the component c1) being selected from acrylic acid, methacrylic acid, itaconic acid and mixtures thereof.

7. The copolymer according to any of the preceding claims, the component c2) being selected from esters of α,β-ethylenically unsaturated mono- and dicarboxylic acids with amino alcohols which may be mono- and dialkylated at the amine nitrogen, amides of α,β-ethylenically unsaturated mono- and dicarboxylic acids with diamines which have at least one primary or secondary amino group, N,N-diallylamine, N,N-diallyl-N-alkylamines and the derivatives thereof, vinyl- and allyl-substituted nitrogen heterocycles, vinyl- and allyl-substituted heteroaromatic compounds and mixtures thereof.

8. The copolymer according to any of the preceding claims, the component c2) being selected from N-vinylimidazole, N-(tert-butylamino)ethyl (meth)acrylate, N,N-dimethylaminoethyl (meth)acrylate, N-[3-(dimethylamino)propyl]-(meth)acrylamide and mixtures thereof.

9. The copolymer according to any of the preceding claims, which additionally comprises, as component d), at least one N-vinyllactam incorporated in the form of polymerized units.

10. The copolymer according to any of the preceding claims, which additionally comprises, incorporated in the form of polymerized units, at least one further monomer e) which is selected from esters, differing from component a), of α,β-ethylenically unsaturated mono- and dicarboxylic acids with C₁-C₃₀-alkanols and C₁-C₃₀-alkanediols, amides of α,β-ethylenically unsaturated mono- and dicarboxylic acids with C₂-C₃₀-amino alcohols which have a primary or secondary amino group, N-vinylamides of saturated monocarboxylic acids, primary amides of α,β-ethylenically unsaturated monocarboxylic acids and the N-alkyl and N,N-dialkyl derivatives thereof, esters of vinyl alcohol and allyl alcohol with C₁-C₃₀-monocarboxylic acids, vinyl ethers, vinylaromatics, vinyl halides, vinylidene halides, C₁-C₈-monoolefins, nonaromatic hydrocarbons having at least two conjugated double bonds and mixtures thereof.

11. The copolymer according to claim 10, the component e) being selected from C₁-C₃-alkyl methacrylates, hydroxy-C₁-C₃-alkyl methacrylates and mixtures thereof.

12. The copolymer according to claim 11, the component e) comprising or consisting of ethyl methacrylate.

13. The copolymer according to any of the preceding claims, which comprises
- from 20 to 90, particularly preferably from 25 to 85, % by weight of at least one compound a),
- from 5 to 40, particularly preferably from 10 to 35, in particular from 13 to 30, % by weight of acrylic acid and/or methacrylic acid b),
- from 0.5 to 25, particularly preferably from 1 to 20, in particular from 2 to 16, % by weight of a monomer composition c),
- from 0 to 85, particularly preferably from 1 to 60, in particular from 5 to 50, % by weight of at least one compound d),
- from 0 to 25, particularly preferably from 0.1 to 20, in particular from 1 to 15, % by weight of at least one compound e),
- from 0 to 5, particularly preferably from 0.01 to 3, in particular from 0.1 to 2, % by weight of at least one crosslinking agent f),
incorporated in the form of polymerized units.

14. The copolymer according to any of claims 1 to 13, which consists of repeating units of
- tert-butyl acrylate,
- acrylic acid and/or methacrylic acid
- N-(tert-butyl)aminoethyl (meth)acrylate or N-[3-(dimethylamino)propyl]-methacrylamide or N,N-dimethylaminoethyl methacrylate or N-vinylimidazole.

15. The copolymer according to any of claims 1 to 13, which consists of repeating units of
- tert-butyl acrylate,
- tert-butyl methacrylate,
- acrylic acid and/or methacrylic acid
- N-(tert-butyl)aminoethyl (meth)acrylate or N-[3-(dimethylamino)propyl]-methacrylamide or N,N-dimethylaminoethyl methacrylate or N-vinylimidazole.

16. The copolymer according to any of claims 1 to 13, which consists of repeating units of
- tert-butyl acrylate,
- acrylic acid and/or methacrylic acid,
- N-(tert-butyl)acrylamide and
- N-(tert-butyl)aminoethyl (meth)acrylate or N-[3-(dimethylamino)propyl]-methacrylamide or N,N-dimethylaminoethyl methacrylate or N-vinylimidazole.

17. The copolymer according to any of claims 1 to 13, which consists of repeating units of
- tert-butyl acrylate,
- acrylic acid and/or methacrylic acid,
- vinylpyrrolidone and
- N-(tert-butyl)aminoethyl (meth)acrylate or N-[3-(dimethylamino)propyl]-methacrylamide or N,N-dimethylaminoethyl methacrylate or N-vinylimidazole.

18. The copolymer according to any of claims 1 to 13, which consists of repeating units of
- tert-butyl acrylate,
- acrylic acid and/or methacrylic acid,
- N-(tert-butyl)acrylamide,
- vinylpyrrolidone and
- N-(tert-butyl)aminoethyl (meth)acrylate or N-[3-(dimethylamino)propyl]-methacrylamide or N,N-dimethylaminoethyl methacrylate or N-vinylimidazole.

19. The copolymer according to any of claims 1 to 13, which comprises, incorporated in the form of polymerized units,
a) tert-butyl acrylate,
b) acrylic acid and/or methacrylic acid,
c) N-(tert-butyl)aminoethyl acrylate or N-(tert-butyl)aminoethyl (meth)acrylate or N-[3-(dimethylamino)propyl]methacrylamide or N,N-dimethylaminoethyl methacrylate or N-vinylimidazole,
d) if appropriate, vinylpyrrolidone and
e) at least one compound which is selected from C₁-C₃-alkyl methacrylates, hydroxy-C₁-C₃-alkyl methacrylates and mixtures thereof,
with the proviso that the proportion by weight of component a) is equal to or greater than the proportion by weight of component e).

20. The copolymer according to any of claims 1 to 13, which consists of repeating units of
- tert-butyl acrylate,
- acrylic acid and/or methacrylic acid,
- N-(tert-butyl)aminoethyl (meth)acrylate or N-vinylimidazole and
- ethyl methacrylate,
with the proviso that the proportion by weight of tert-butyl acrylate is equal to or greater than the proportion by weight of ethyl methacrylate.

21. The copolymer according to any of claims 1 to 13, which consists of repeating units of
- tert-butyl acrylate,
- acrylic acid and/or methacrylic acid,
- N-vinylpyrrolidone,
- N-(tert-butyl)aminoethyl (meth)acrylate or N-vinylimidazole and
- ethyl methacrylate,
with the proviso that the proportion by weight of tert-butyl acrylate is equal to or greater than the proportion by weight of ethyl methacrylate.

22. The copolymer according to any of claims 1 to 13, which comprises, based in each case on the total weight of the monomers used for the polymerization,
- from 20 to 80% by weight, preferably from 25 to 75% by weight, of tert-butyl acrylate and ethyl methacrylate, with the proviso that the proportion by weight of tert-butyl acrylate is equal to or greater than the proportion by weight of ethyl methacrylate,
- from 5 to 30% by weight, preferably from 10 to 25% by weight, of acrylic acid and/or methacrylic acid,
- from 1 to 20% by weight, preferably from 3 to 15% by weight, of a monomer composition comprising methacrylic acid and N-vinylimidazole or N-(tert-butyl)aminoethyl (meth)acrylate and
- from 0 to 35% by weight of vinylpyrrolidone,
incorporated in the form of polymerized units.

23. A polyelectrolyte complex comprising at least one ampholytic copolymer, as defined in any of claims 1 to 22, and at least one polyelectrolyte PE) differing therefrom.

24. A cosmetic or pharmaceutical composition comprising
A) at least one ampholytic copolymer, as defined in any of claims 1 to 22, or a polyelectrolyte complex, as defined in claim 23, and
B) at least one cosmetically acceptable carrier.

25. The composition according to claim 24, the component B) being selected from
i) water,
ii) water-miscible organic solvents, preferably C₂-C₄-alkanols, in particular ethanol,
iii) oils, fats, waxes,
iv) esters of C₆-C₃₀-monocarboxylic acids with monohydric, dihydric or trihydric alcohols, which esters differ from iii),
v) saturated acyclic and cyclic hydrocarbons,
vi) fatty acids,
vii) fatty alcohols,
viii) propellants
and mixtures thereof.

26. The composition according to either of claims 24 and 25, comprising at least one additive which differs from the components A) and B) and is selected from cosmetically active substances, emulsifiers, surfactants, preservatives, perfume oils, thickeners, hair polymers, hair and skin conditioners, graft polymers, watersoluble or dispersible silicone-containing polymers, light stabilizers, bleaches, gel formers, care compositions, colorants, tinting compositions, tanning compositions, dyes, pigments, consistency agents, moisturizers, refatting agents, collagen, protein hydrolysis products, lipids, antioxidants, antifoams, antistatic agents, emollients and plasticizers.

27. The composition according to any of claims 24 to 26 in the form of a gel, of a foam, of a spray or of a mousse, ointment, cream, emulsion, suspension, lotion, milk or paste.

28. The use of a copolymer, as defined in any of claims 1 to 22, or of a polyelectrolyte complex, as defined in claim 23, in skin cleansing compositions, compositions for the care and for the protection of the skin, nail care compositions, formulations for decorative cosmetics and hair treatment compositions.

29. The use according to claim 28 in hair treatment compositions as setting compositions and/or as conditioners.

30. The use according to claim 29, the composition being present in the form of a hair gel, shampoo, foam setting composition, hair lotion, hairspray or hair foam.

31. The use of a copolymer, as defined in any of claims 1 to 22, or of a polyelectrolyte complex, as defined in claim 23, as an assistant in pharmacy, preferably as or in coating material(s) for solid dosage forms, for modifying rheological properties, as a surface-active compound, as or in adhesive(s) and as or in coating material(s) for the textile, paper, printing and leather industry.

## Revendications

1. Copolymère ampholytique, contenant un excès molaire de groupes anionogènes et/ou anioniques, pouvant être obtenu par polymérisation par voie radicalaire
a) d'au moins un acrylate de C₃-C₅-alkyle ramifié,
b) d'acide méthacrylique et/ou d'acide méthacrylique et
c) d'au moins une composition monomère, contenant
c1) au moins un composé avec une double liaison α,ß-éthyléniquement insaturée, polymérisable par voie radicalaire et au moins un groupe anionogène et/ou anionique par molécule et
c2) au moins un composé avec une double liaison α,β-éthyléniquement insaturée, polymérisable par voie radicalaire et au moins un groupe cationogène et/ou cationique par molécule,
le rapport molaire des groupes anionogènes et anioniques du composant c1) aux groupes cationogènes et cationiques du composant c2) étant d'environ 1:1.

2. Copolymère selon la revendication 1, le composant a) comprenant de l'acrylate de tert-butyle.

3. Copolymère selon la revendication 1, où le composant b) est constitué d'acide acrylique et d'acide méthacrylique, à condition que la proportion pondérale d'acide acrylique soit identique ou supérieure à la proportion pondérale d'acide méthacrylique.

4. Copolymère selon la revendication 1, où le composant b) est constitué d'acide acrylique ou d'acide méthacrylique.

5. Copolymère selon l'une quelconque des revendications précédentes, le composant c1) étant choisi parmi les acides monocarboxyliques et dicarboxyliques α,β-éthyléniquement insaturés, les acides sulfoniques, les acides phosphoniques et leurs mélanges.

6. Copolymère selon l'une quelconque des revendications précédentes, le composant c1) étant choisi parmi l'acide acrylique, l'acide méthacrylique, l'acide itaconique et leurs mélanges.

7. Copolymère selon l'une quelconque des revendications précédentes, le composant c2) étant choisi parmi les esters d'acides monocarboxyliques et dicarboxyliques α,β-éthyléniquement insaturés avec des aminoalcools, qui peuvent être monoalkylés ou dialkylés sur l'azote de la fonction amine, les amides d'acides monocarboxyliques et dicarboxyliques α,β-éthyléniquement insaturés avec des diamines qui présentent au moins un groupe amino primaire ou secondaire, la N,N-diallylamine, les N,N-diallyl-N-alkylamines et leurs dérivés, les hétérocycles azotés substitués par vinyle et allyle, les composés hétéroaromatiques substitués par vinyle et allyle et leurs mélanges.

8. Copolymère selon l'une quelconque des revendications précédentes, le composant c2) étant choisi parmi le N-vinylimidazole, le (méth)acrylate de N-(tert-butylamino)éthyle, le (méth)acrylate de N,N-diméthylaminoéthyle, le N-[3-(diméthylamino)propyl]-(méth)acrylamide et leurs mélanges.

9. Copolymère selon l'une quelconque des revendications précédentes, qui contient en outre, comme composant d), au moins un N-vinyllactame sous forme copolymérisée.

10. Copolymère selon l'une quelconque des revendications précédentes, qui contient en outre au moins un autre monomère e) sous forme copolymérisée, qui est choisi parmi les esters, différents du composant a), d'acides monocarboxyliques et dicarboxyliques α,β-éthyléniquement insaturés avec des C₁-C₃₀-alcanols et des C₁-C₃₀-alcanediols, les amides d'acides monocarboxyliques et dicarboxyliques α,β-éthyléniquement insaturés avec des C₂-C₃₀-aminoalcools, qui présentent un groupe amino primaire ou secondaire, les N-vinylamides d'acides monocarboxyliques saturés, les amides primaires d'acides monocarboxyliques α,β-éthyléniquement insaturés et leurs dérivés de N-alkyle et N,N-dialkyle, les esters de l'alcool vinylique et de l'alcool allylique avec des acides monocarboxyliques en C₁-C₃₀, les vinyléthers, les aromatiques de vinyle, les halogénures de vinyle, les halogénures de vinylidène, les monooléfines en C₁-C₈, les hydrocarbures non aromatiques avec au moins deux doubles liaisons conjuguées et leurs mélanges.

11. Copolymère selon la revendication 10, le composant e) étant choisi parmi les méthacrylates de C₁-C₃-alkyle, les méthacrylates d'hydroxy-C₁-C₃-alkyle et leurs mélanges.

12. Copolymère selon la revendication 11, le composant e) comprenant du méthacrylate d'éthyle ou étant constitué par celui-ci.

13. Copolymère selon l'une quelconque des revendications précédentes qui contient, sous forme copolymérisée
- 20 à 90% en poids, de manière particulièrement préférée 25 à 85% en poids, d'au moins un composé a),
- 5 à 40% en poids, de manière particulièrement préférée 10 à 35% en poids, en particulier 13 à 30% en poids d'acide acrylique et/ou d'acide méthacrylique b),
- 0,5 à 25% en poids, de manière particulièrement préférée 1 à 20% en poids, en particulier 2 à 16% en poids d'une composition monomère c),
- 0 à 85% en poids, de manière particulièrement préférée 1 à 60% en poids, en particulier 5 à 50% en poids, d'au moins un composé d),
- 0 à 25% en poids, de manière particulièrement préférée 0,1 à 20% en poids, en particulier 1 à 15% en poids, d'au moins un composé e),
- 0 à 5% en poids, de manière particulièrement préférée 0,01 à 3% en poids, en particulier 0,1 à 2% en poids, d'au moins un réticulant f).

14. Copolymère selon l'une quelconque des revendications 1 à 13, qui est constitué par des unités répétitives
- d'acrylate de tert-butyle,
- d'acide acrylique et/ou d'acide méthacrylique
- de (méth)acrylate de N-(tert-butyl)aminoéthyle ou de N-[3-(diméthylamino)propyl]méthacrylamide ou de méthacrylate de N,N-diméthylaminoéthyle ou de N-vinylimidazole.

15. Copolymère selon l'une quelconque des revendications 1 à 13, qui est constitué par des unités répétitives
- d'acrylate de tert-butyle,
- de méthacrylate de tert-butyle,
- d'acide acrylique et/ou d'acide méthacrylique,
- de (méth)acrylate de N-(tert-butyl)aminoéthyle ou de N-[3-(diméthylamino)propyl]méthacrylamide ou de méthacrylate de N,N-diméthylaminoéthyle ou de N-vinylimidazole.

16. Copolymère selon l'une quelconque des revendications 1 à 13, qui est constitué par des unités répétitives
- d'acrylate de tert-butyle,
- d'acide acrylique et/ou d'acide méthacrylique,
- de N-(tert-butyl)acrylamide et
- de (méth)acrylate de N-(tert-butyl)aminoéthyle ou de N[3-(diméthylamino)propyl]méthacrylamide ou de méthacrylate de N,N-diméthylaminoéthyle ou de N-vinylimidazole.

17. Copolymère selon l'une quelconque des revendications 1 à 13, qui est constitué par des unités répétitives
- d'acrylate de tert-butyle,
- d'acide méthacrylique et/ou d'acide méthacrylique,
- de vinylpyrrolidone et
- de (méth)acrylate de N-(tert-butyl)aminoéthyle ou de N[3-(diméthylamino)propyl]méthacrylamide ou de méthacrylate de N,N-diméthylaminoéthyle ou de N-vinylimidazole.

18. Copolymère selon l'une quelconque des revendications 1 à 13, qui est constitué par des unités répétitives
- d'acrylate de tert-butyle,
- d'acide acrylique et/ou d'acide méthacrylique,
- de N-(tert-butyl)acrylamide,
- de vinylpyrrolidone et
- de (méth)acrylate de N-(tert-butyl)aminoéthyle ou de N-[3-(diméthylamino)propyl]méthacrylamide ou de méthacrylate de N,N-diméthylaminoéthyle ou de N-vinylimidazole.

19. Copolymère selon l'une quelconque des revendications 1 à 13, qui contient, sous forme copolymérisée
a) de l'acrylate de tert-butyle,
b) de l'acide acrylique et/ou de l'acide méthacrylique,
c) de l'acrylate de N-(tert-butyl)aminoéthyle ou du (méth)acrylate de N-(tert-butyl)aminoéthyle ou du N[3-(diméthylamino)-propyl]méthacrylamide ou du méthacrylate de N,N-diméthylaminoéthyle ou du N-vinylimidazole
d) le cas échéant de la vinylpyrrolidone et
e) au moins un composé qui est choisi parmi les méthacrylates de C₁-C₃-alkyle, les méthacrylates d'hydroxy-C₁-C₃-alkyle et leurs mélanges
à condition que la proportion pondérale du composant a) soit identique ou supérieure à la proportion pondérale du composant e).

20. Copolymère selon l'une quelconque des revendications 1 à 13, qui est constitué par des unités répétitives
- d'acrylate de tert-butyle,
- d'acide acrylique et/ou d'acide méthacrylique,
- de (méth)acrylate de N-(tert-butyl)aminoéthyle ou de N-vinylimidazole et
- de méthacrylate d'éthyle
à condition que la proportion pondérale du composant d'acrylate de tert-butyle soit identique ou supérieure à la proportion pondérale de méthacrylate d'éthyle.

21. Copolymère selon l'une quelconque des revendications 1 à 13, qui est constitué par des unités répétitives
- d'acrylate de tert-butyle,
- d'acide acrylique et/ou d'acide méthacrylique,
- de N-vinylpyrrolidone,
- de (méth)acrylate de N-(tert-butyl)aminoéthyle ou de N-vinylimidazole et
- de méthacrylate d'éthyle
à condition que la proportion pondérale du composant d'acrylate de tert-butyle soit identique ou supérieure à la proportion pondérale de méthacrylate d'éthyle.

22. Copolymère selon l'une quelconque des revendications 1 à 13, qui contient sous forme copolymérisée, à chaque fois par rapport au poids total des monomères utilisés pour la polymérisation,
- 20 à 80% en poids, de préférence 25 à 75% en poids, d'acrylate de tert-butyle et de méthacrylate d'éthyle, à condition que la proportion pondérale d'acrylate de tert-butyle soit identique ou supérieure à la proportion pondérale de méthacrylate d'éthyle,
- 5 à 30% en poids, de préférence 10 à 25% en poids d'acide acrylique et/ou d'acide méthacrylique,
- 1 à 20% en poids, de préférence 3 à 15% en poids d'une composition monomère d'acide méthacrylique et de N-vinylimidazole ou de (méth)acrylate de N-(tert-butyl)aminoéthyle et
- 0 à 35% en poids de vinylpyrrolidone.

23. Complexe polyélectrolytique, contenant au moins un copolymère ampholytique, tel que défini dans les revendications 1 à 22, et au moins un polyélectrolyte PE différent de celui-ci.

24. Agent cosmétique ou pharmaceutique, contenant
A) au moins un copolymère ampholytique, tel que défini dans les revendications 1 à 22 ou un complexe polyélectrolytique, tel que défini dans la revendication 23, et
B) au moins un support cosmétiquement acceptable.

25. Agent selon la revendication 24, où le composant B) est choisi parmi
i) l'eau,
ii) les solvants organiques miscibles à l'eau, de préférence les C₂-C₄-alcanols, en particulier l'éthanol
iii) les huiles, les graisses, les cires,
iv) les esters différents de iii) d'acides monocarboxyliques en C₆-C₃₀ avec des alcools monovalents, divalents ou trivalents,
v) les hydrocarbures acycliques et cycliques saturés,
vi) les acides gras,
vii) les alcools gras,
viii) les gaz propulseurs
et leurs mélanges.

26. Agent selon l'une quelconque des revendications 24 ou 25, contenant au moins un additif différent des composants A) et B), qui est choisi parmi les substances cosmétiquement actives, les émulsifiants, les agents tensioactifs, les conservateurs, les huiles parfumées, les épaississants, les polymères pour cheveux, les agents revitalisants pour les cheveux et la peau, les polymères greffés, les polymères siliconés solubles ou dispersibles dans l'eau, les agents de protection contre la lumière, les agents de blanchiment, les gélifiants, les agents de soin, les agents de teinture, les agents de nuançage, les agents de bronzage, les colorants, les pigments, les agents conférant une consistance, les humidifiants, les relipidants, le collagène, les hydrolysats de protéines, les lipides, les antioxydants, les antimousses, les antistatiques, les émollients et les plastifiants.

27. Agent selon l'une quelconque des revendications 24 à 26 sous forme d'un gel, d'une mousse, d'un spray, d'une pommade, d'une crème, d'une émulsion, d'une suspension, d'une lotion, d'un lait ou d'une pâte.

28. Utilisation d'un copolymère tel que défini dans l'une quelconque des revendications 1 à 22 ou d'un complexe polyélectrolytique, tel que défini dans la revendication 23, dans des agents de nettoyage de la peau, des agents pour le soin et la protection de la peau, les agents de soin des ongles, les compositions pour la cosmétique décorative et les agents de traitement des cheveux.

29. Utilisation selon la revendication 28 dans les agents de traitement de cheveux comme fixateur et/ou comme agent revitalisant.

30. Utilisation selon la revendication 29, l'agent se trouvant sous forme d'un gel pour cheveux, d'un shampooing, d'un fixateur en mousse, d'une lotion pour cheveux, d'un spray pour cheveux ou d'une mousse pour cheveux.

31. Utilisation d'un copolymère, tel que défini selon l'une quelconque des revendications 1 à 22, ou d'un complexe polyélectrolytique, tel que défini dans la revendication 23, comme adjuvant dans la pharmacie, de préférence comme ou dans le ou les agents de revêtement pour des formes médicamenteuses solides, pour la modification de propriétés rhéologiques, comme composé tensioactif, comme ou dans les agents adhésifs, ainsi que comme ou dans des agents de revêtement pour l'industrie des textiles, des papiers, d'imprimerie et du cuir.
